# EUROPEAN PATENT APPLICATION

(11) **EP 2 065 039 A1**
(43) Date of publication of application: **03.06.2009**
(21) Application number: 07022996.8
(22) Date of filing: 27.11.2007
(51) Int. Cl.: A61K 31/122, C07C 50/04, C07C 50/24, C07C 251/22, C07C 251/46, C07C 251/84, A61K 31/15, C07C 251/62

(54) **Selective inhibition of Polo-like kinase 1**

(71) Applicant: Max-Planck-Gesellschaft zur Förderung der Wissenschaften e.V., 80539 München (DE)
(72) Inventor: Berg, Thorsten, 81475 München (DE); Reindl, Wolfgang, 81373 München (DE); Strebhardt, Klaus, D-61462 Königstein (DE); Yuan, Juping, 61118 Bad Vilbel (DE)
(74) Representative: Arth, Hans-Lothar

(57) **Abstract**

The present invention relates to compounds having a 1,4-benzoquinone or 1,4-benzoquinone imine scaffold, pharmaceutically acceptable salts of these compounds and pharmaceutical compositions containing at least one of these compounds together with pharmaceutically acceptable carrier, excipient and/or diluents. Said 1,4-benzoquinone or 1,4-benzoquinone imine compounds can be used for prophylaxis and/or treatment of cancer and for prophylaxis and/or treatment of inflammatory diseases.

## Description

The present invention relates to 1,4-benzoquinone and 1,4-benzoquinone imine derivatives and stereoisomeric forms, prodrugs, solvates, hydrates and/or pharmaceutically acceptable salts of these compounds as well as pharmaceutical compositions containing at least one of these 1,4-benzoquinone or 1,4- benzoquinone imine derivatives together with pharmaceutically acceptable carrier, excipient and/or diluents. Said 1,4-benzoquinone and 1,4- benzoquinone imine derivatives have been identified as specific inhibitors of Polo-like kinase 1 and are useful for prophylaxis and treatment of cancer and for treatment of inflammatory diseases.

### Background of the invention

The polo-box domain (PBD) is a phosphoserine/phosphothreonine binding domain which mediates localization of the anti-cancer target Polo-like kinase 1 (PIk1) to its intracellular anchoring sites. Targeting protein-protein interaction domains are required for correct intracellular localization of protein kinases and thus represent a viable alternative to their inhibition by ligands to their conserved ATP-binding pocket.

PIk1 localizes to its intracellular anchoring sites *via* its carboxyterminal portion containing the PBD, which binds to an S-(pT/pS)-{P/X) motif. Since the PBD is unique to the family of Plks and is essential for Plk functions, the PBD is ideally suited to the exploration of the feasibility of inhibiting a serine/threonine kinase by selectively interfering with its intracellular localization, rather than targeting the conserved ATP-binding site. Moreover, small-molecules targeting the PBD should be useful research tools to decipher the biological role of the Plk1 PBD in mammalian cells.

The serine/threonine kinase Plk1, a regulator of multiple stages of mitotic progression, is overexpressed in many types of human cancers, and has been implicated as an adverse prognostic marker for cancer patients. Its inhibition in cancer cells by small interfering RNA, a Plk1-derived peptide, antisense oligonucleotides, and small-molecule inhibitors of its catalytic activity has been uniformly demonstrated to induce mitotic arrest and apoptosis of cancer cells *in vitro* and *in vivo.*

To investigate the role of Plk1 in neoplastic proliferation several cancer cell lines were transfected with small interfering RNAs (siRNA) targeted against human Plk1 genes. An important result of these studies was that cell cycle arrest and apoptosis are associated with reduced levels of Plk1 protein (Spänkuch-Schmitt et al., Journal of the National Cancer Institute, Vol. 94, No. 24, p.1863 -1877, 2002).

The German publication DE 10 2004 061 503 A1 discloses thiazolidinones and Yuan et al. Cancer Res., 62, p. 4186-4190, 2002 mentions a Plk1-derived peptide as Polo kinase-Inhibitors. Furthermore Spänkuch-Schmitt et al. (Oncogene, 21, p.3162-3171, 2002) used antisense oligonucleotides to inhibit Polo kinase.

None of these prior art documents disclose the use of the claimed 1,4-benzoquinone and/or 1,4-benzoquinone imine derived compounds described in the present application for treatment or prophylaxis of cancer and for treatment of inflammatory diseases.

It is the object of the present invention to provide compounds and/or pharmaceutically acceptable salts thereof which can be used as pharmaceutically active agents, especially for the treatment or prophylaxis of cancer as well as compositions comprising at least one of those compounds and/or pharmaceutically acceptable salts thereof as pharmaceutically active ingredients.

The object of the present invention is solved by the teaching of the independent claims. Further advantageous features, aspects and details of the invention are evident from the dependent claims, the description, and the examples of the present application.

Surprisingly it has been found that the inventive 1,4-benzoquinone and/or 1,4-benzoquinone imine derivatives inhibit the Plk1 PBD leading to chromosome congression defects, mitotic arrest and apoptosis.

### Description of the invention

The novel 1,4-benzoquinone and 1,4-benzoquinone imine derivatives according to the present invention are represented by the following general formula (I): 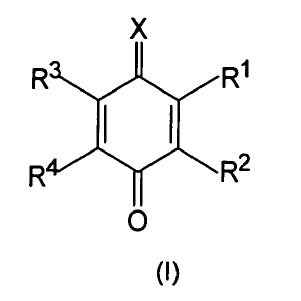 wherein
**R¹ - R⁴** represent independently from each other -H, -R⁵, -R⁶, -R⁷, -R⁸, -CR⁹ R¹⁰ R¹¹, -Y-CR¹²R¹³R¹⁴, -Y-R¹⁵
**Y** represents independently from each other -(CH₂)ₙ-, -O-, -S-, -NH-, -CH=CH-, -C=C-, -O-(CH₂)ₘ-, -NH-(CH₂)ₚ-, -CO-, -CO-O-, -NH-CO-, -CO-NH-;
   wherein n, m and p are independently from each other integers from 1 - 6;
**R⁵ - R¹⁵** represent independently from each other -H, -F, -Cl, -Br, -I, -CH₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH₂OH, -CH₂-CH(Cl)-CH₃, -CH₂-CH(Br)-CH₃, -CH₂-CH(I)-CH₃, -CH₂-CH(OH)-CH₃, -CH₂-CH(NH₂)-CH₃, cyclo-C₃H₅, cyclo-C₄H₇, cyclo-C₅H₉, cyclo-C₆H₁₁, cyclo-C₇H₁₃, cyclo-C₈H₁₅, -Ph, CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁,-CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃,-CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃. -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂,-C₃H₆-CH=CH₂, -C₂H₄-CH=CH-CH₃, -CH₂-CH=CH-CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH-C₂H₅, -CH=CH-C₃H₇, -CH₂-CH=CH-CH=CH₂, -CH=CH-CH=CH-CH₃, -CH=CH-CH₂-CH=CH₂,-C(CH₃)=CH-CH=CH₂. -CH=C(CH₃)-CH=CH₂, (CH₃)-CH=CH-CH₃, -CH=CH-CH(CH₃)₂, -CH=C(CH₃)-C₂H₅, -C(CH₃)=CH-C₂H₅,-C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂. -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂. -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂. -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂. -CH₂--C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C=C-CH₃-CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅. -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, -CH=CH-Ph, -Ph, -CH₂-Ph;
**X** represents =O, =N-OR¹⁶, =N-NHR¹⁶, =N-O-(CO)-R¹⁶, =N-NH-(CO)-R¹⁶;
**R¹⁶** represents -R⁴¹, -CR⁴²R⁴³R⁴⁴, -Z-CR⁴²R⁴³R⁴⁴, -Z-R⁴¹, 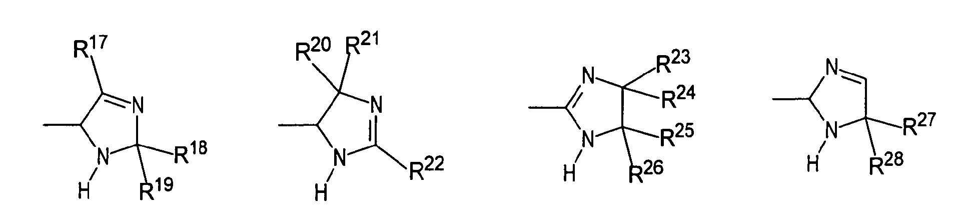 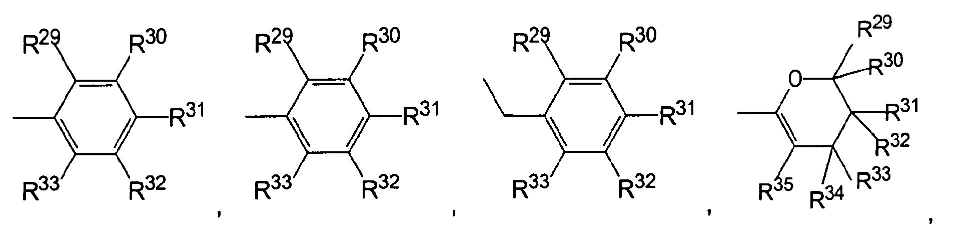 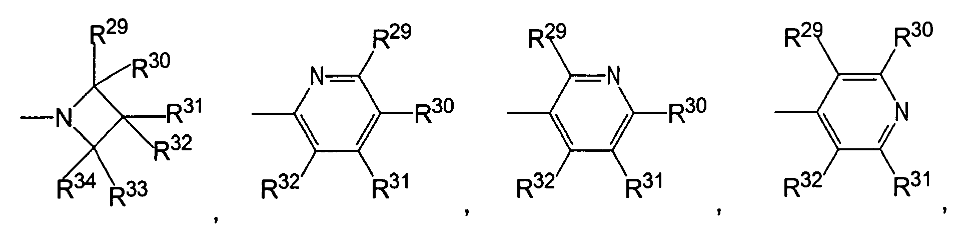 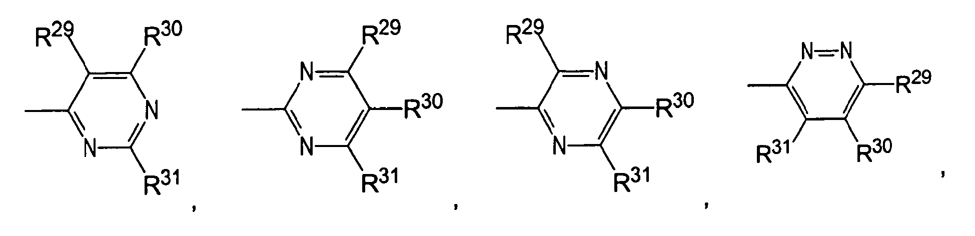 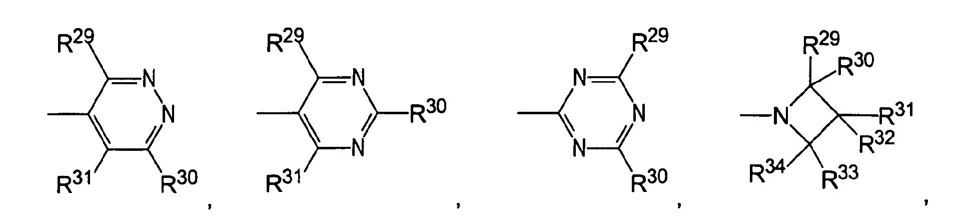 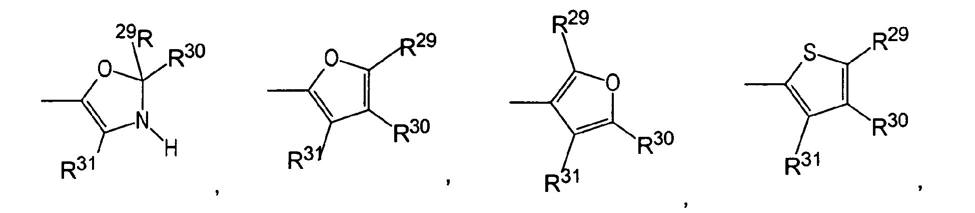 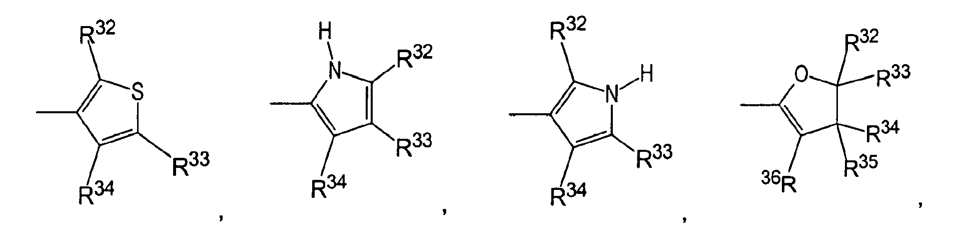 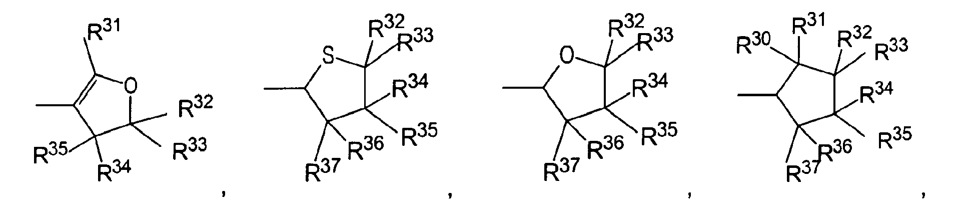 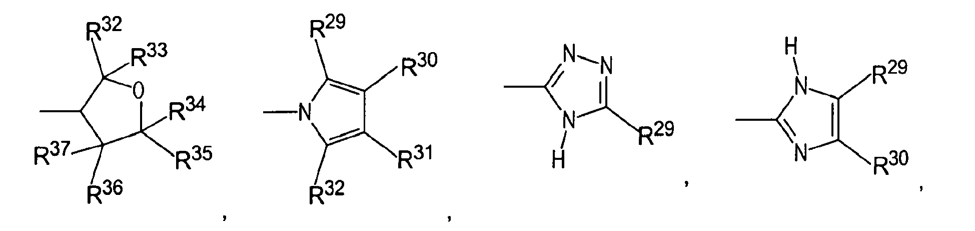 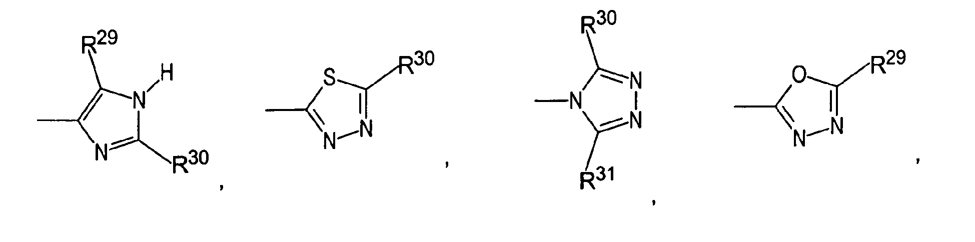 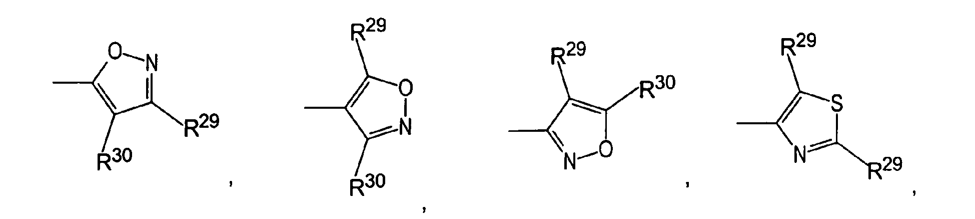 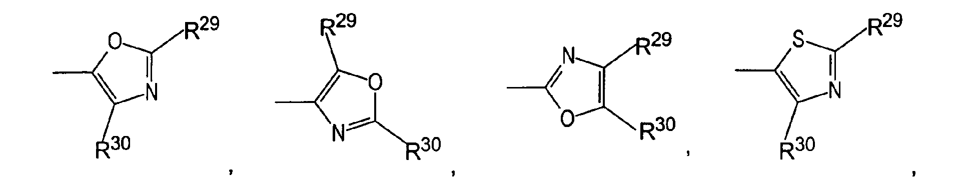 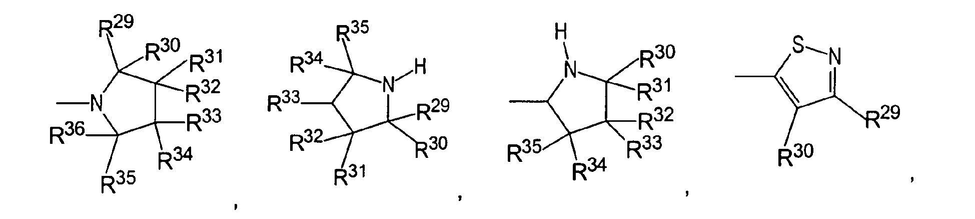 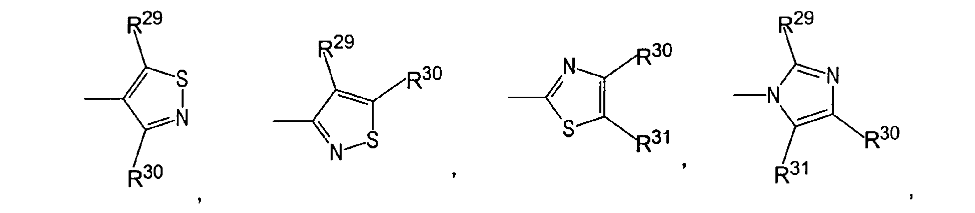 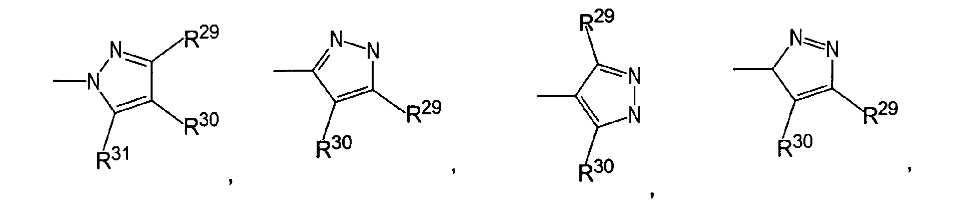 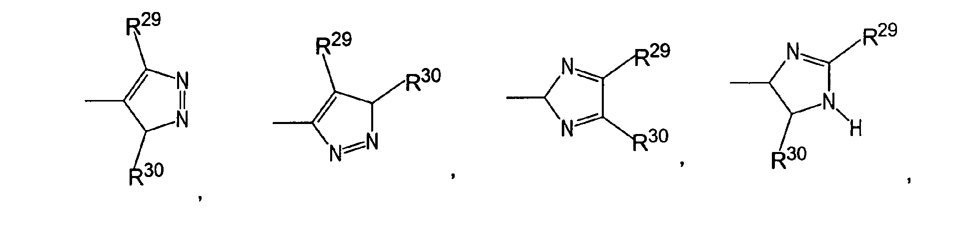 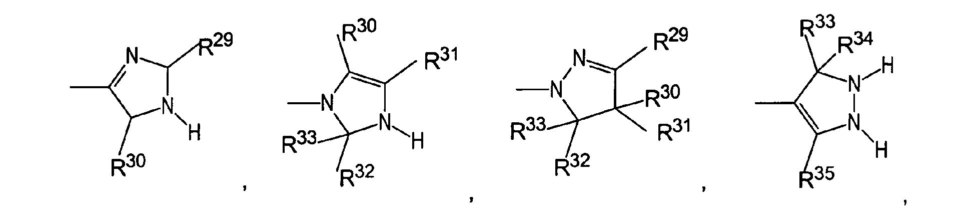 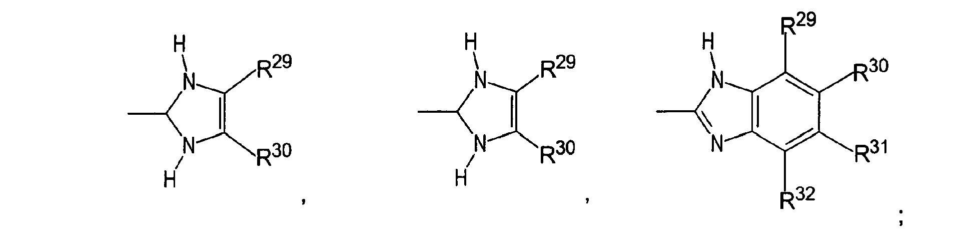 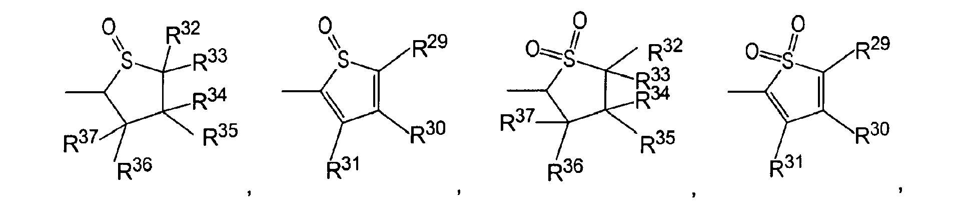 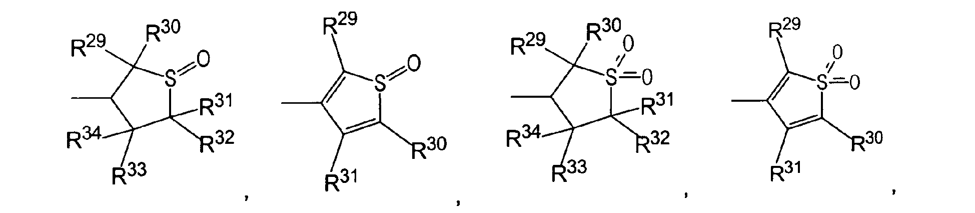 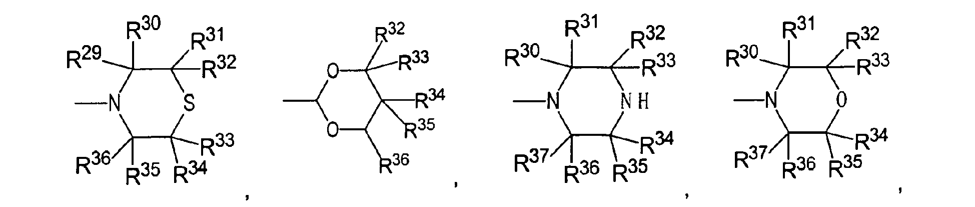 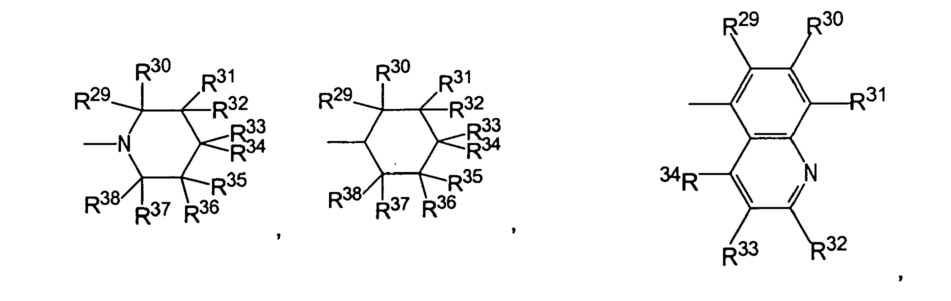 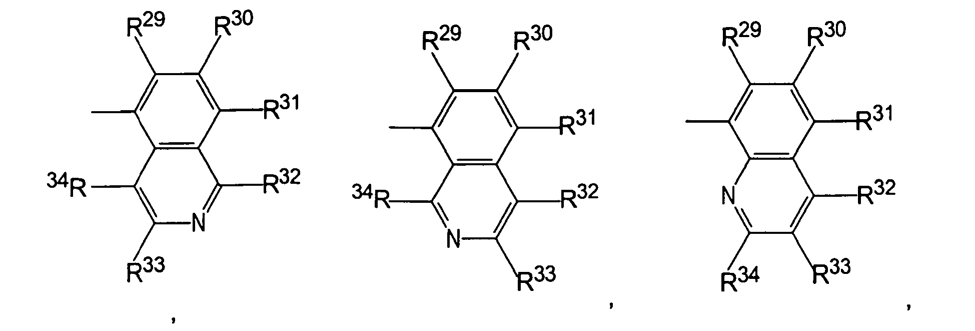 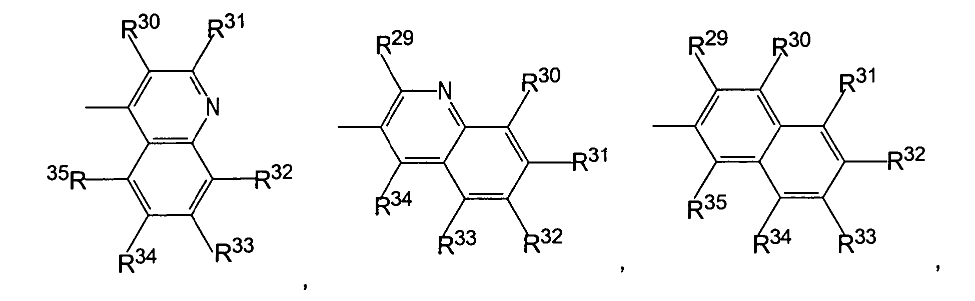 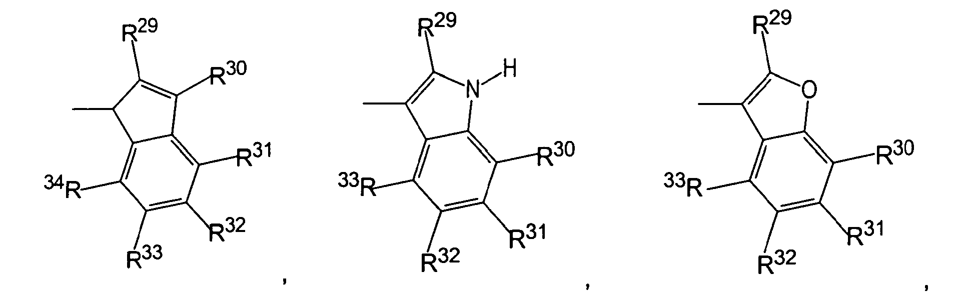 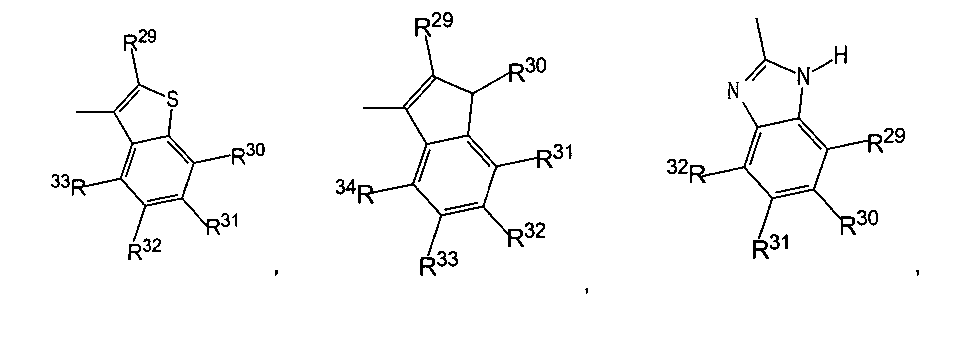 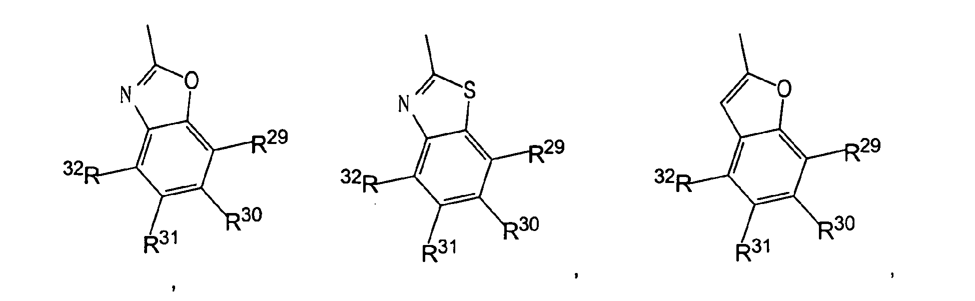 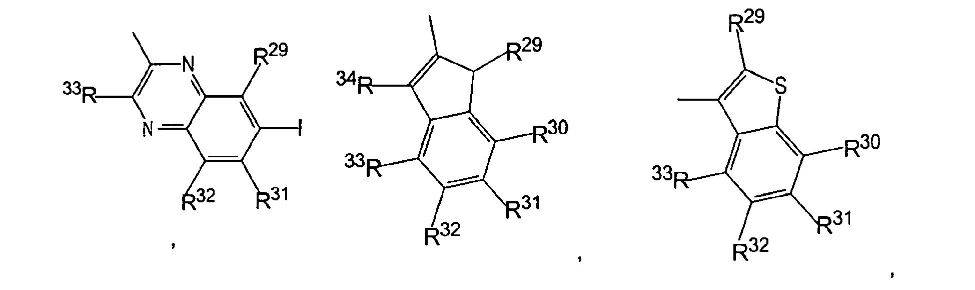 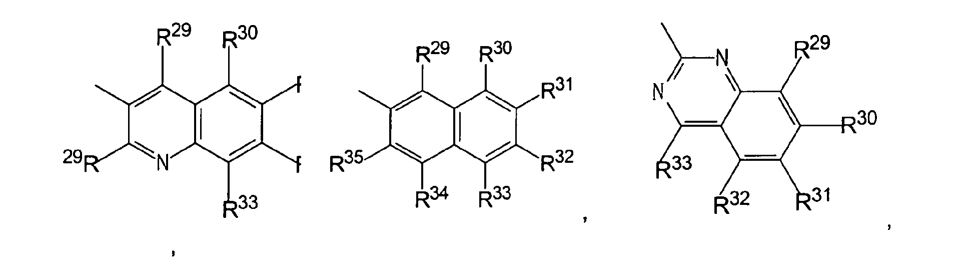 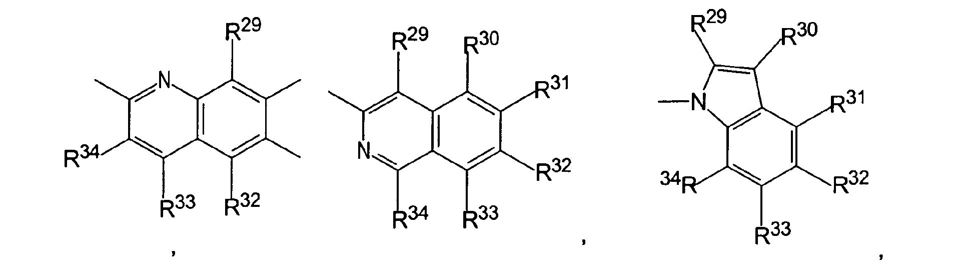 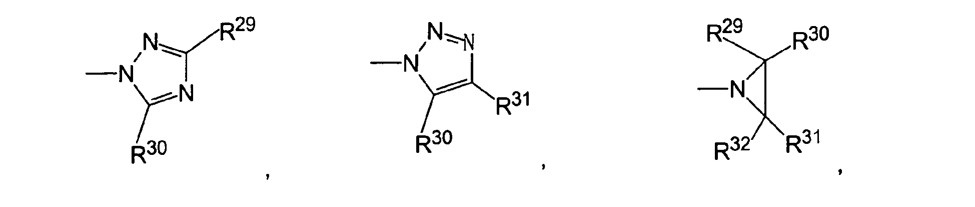 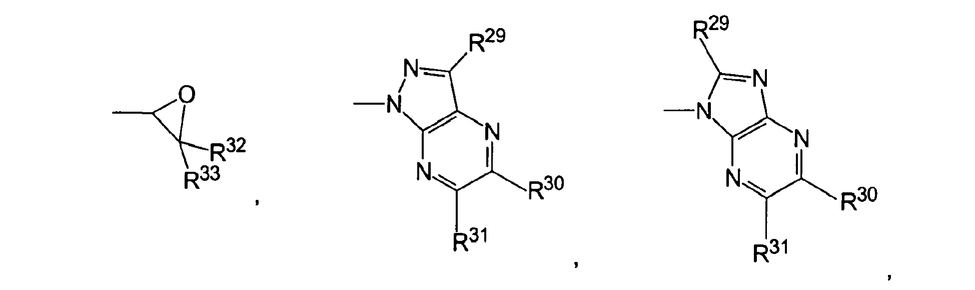 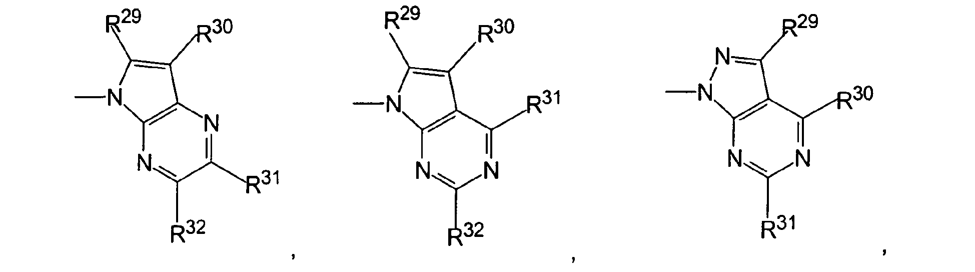 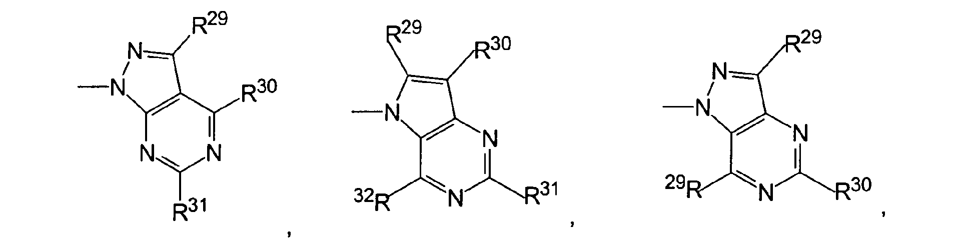 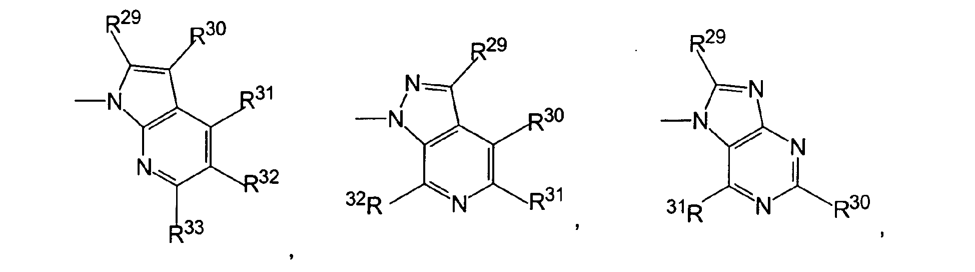 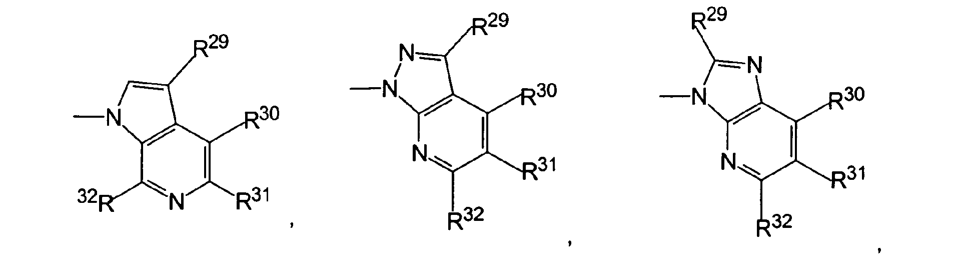 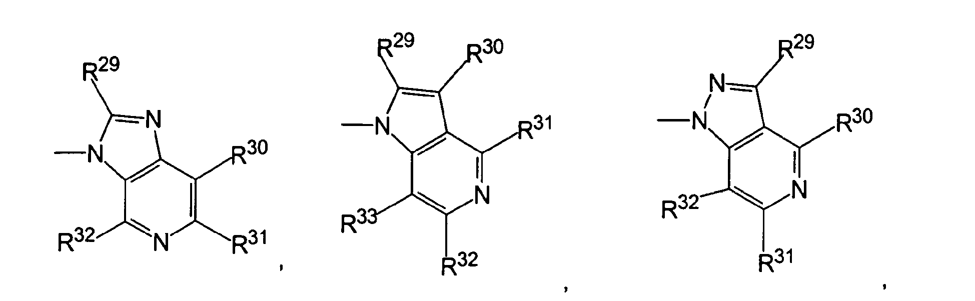 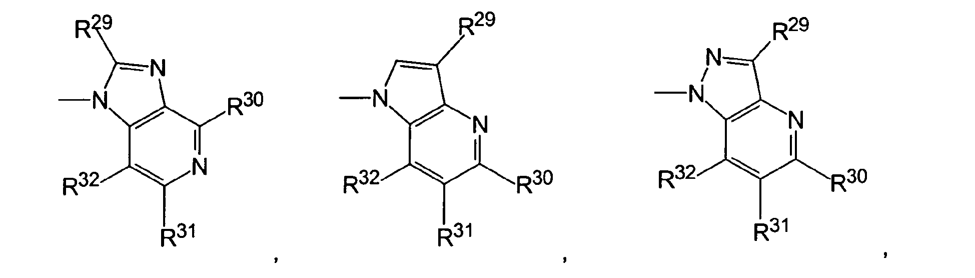 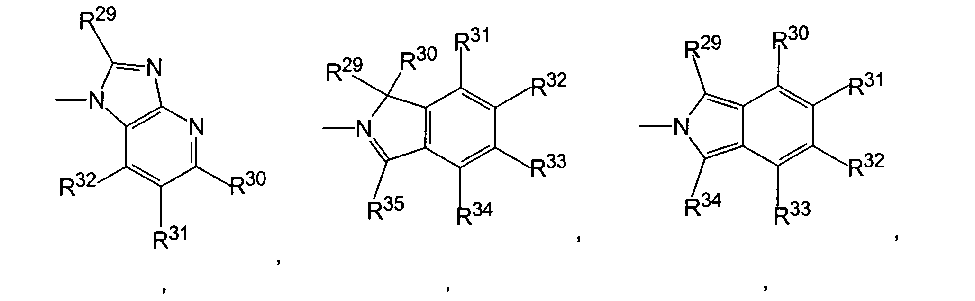 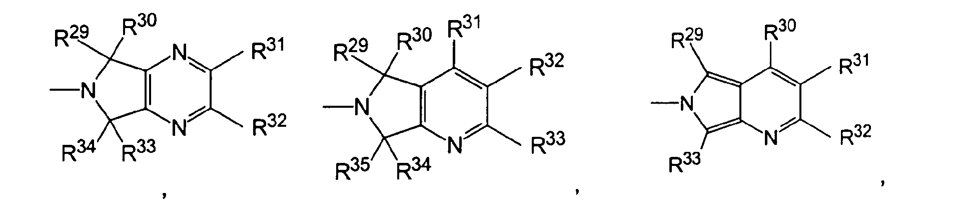 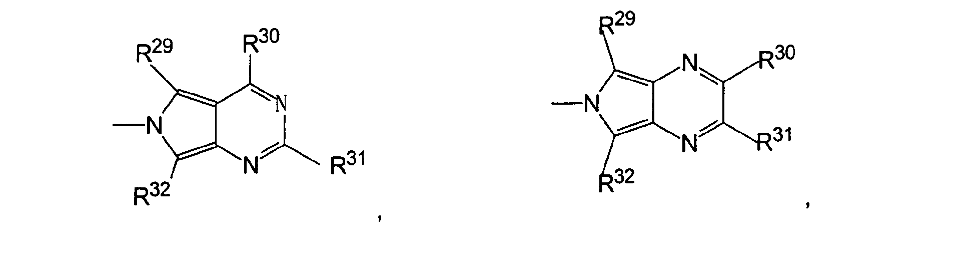 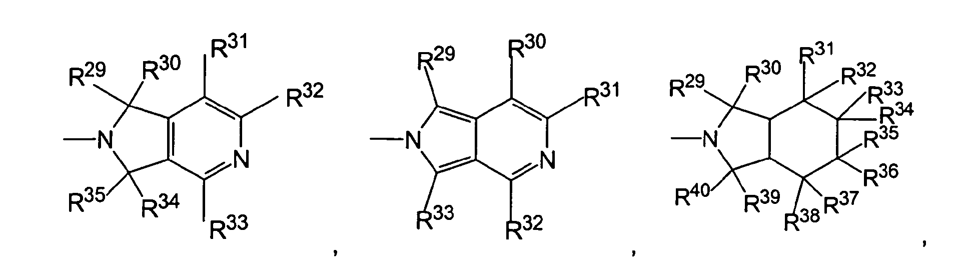 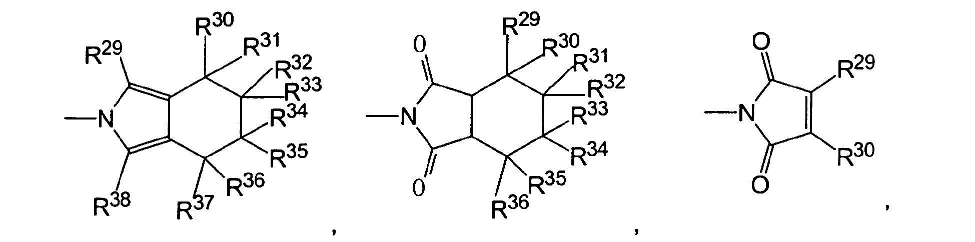 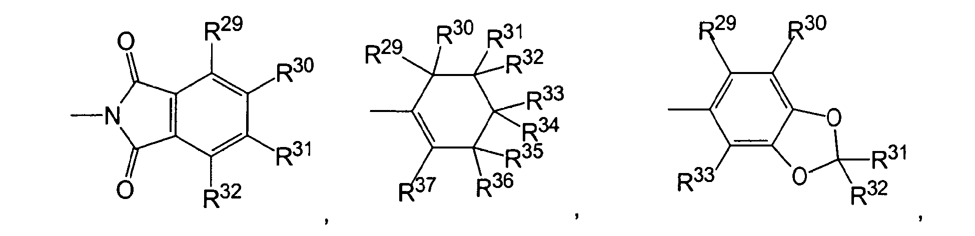 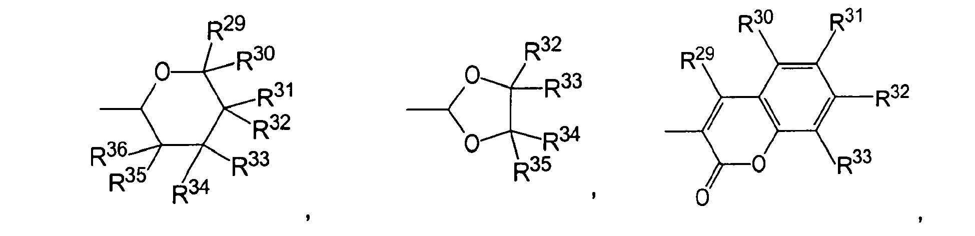 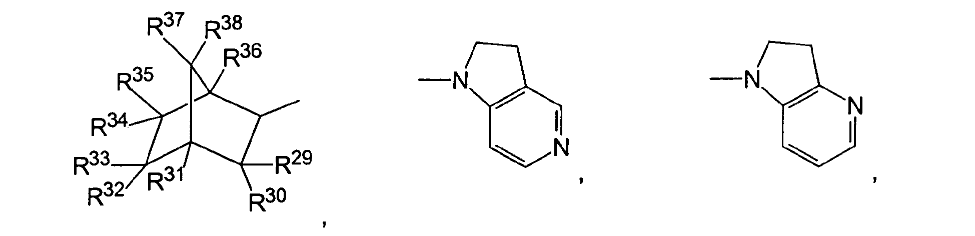 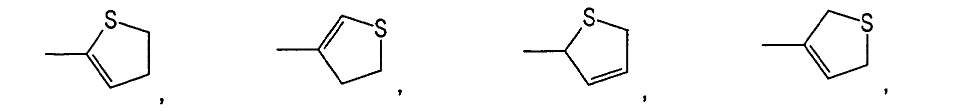 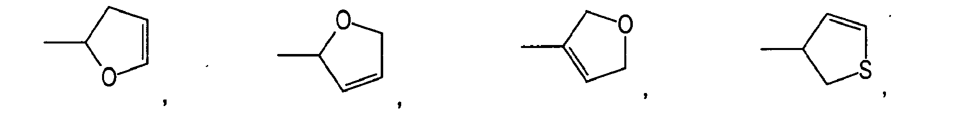 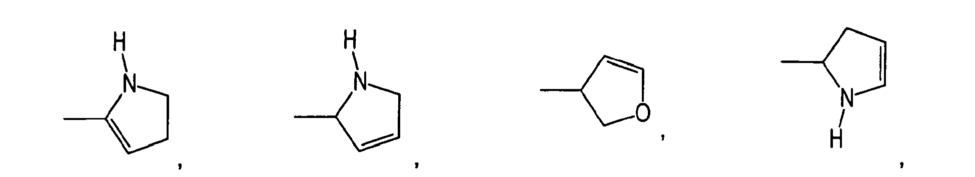 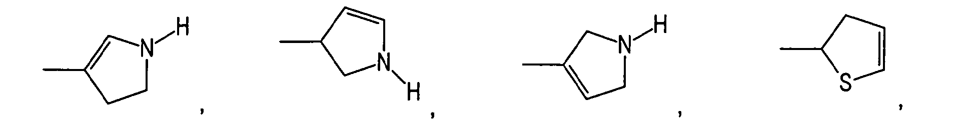 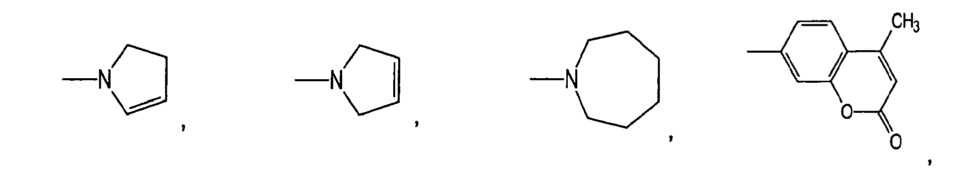 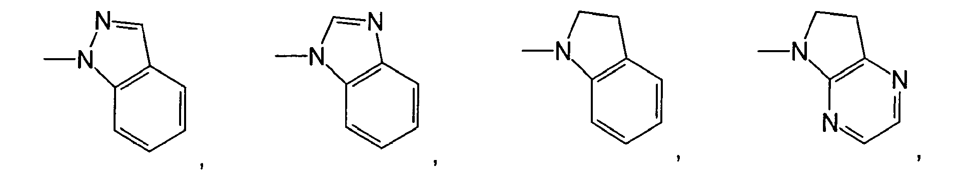 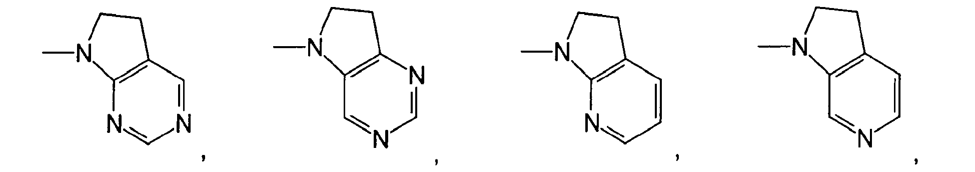 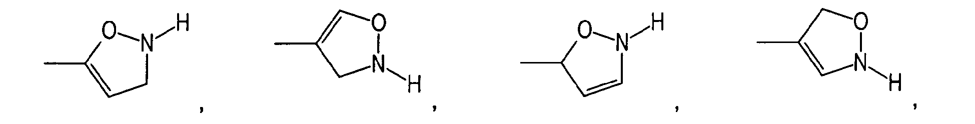 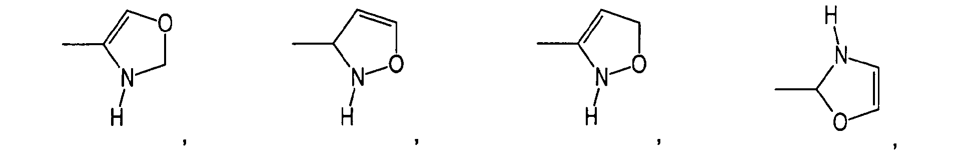 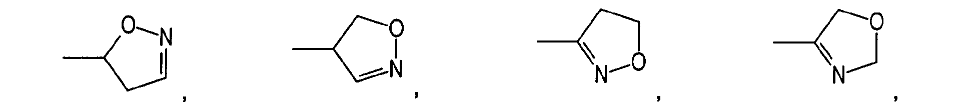 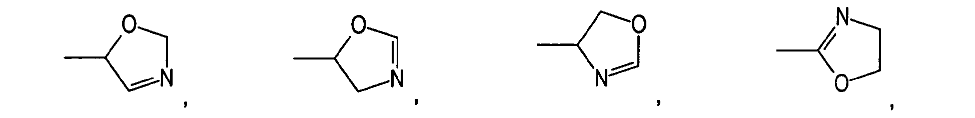 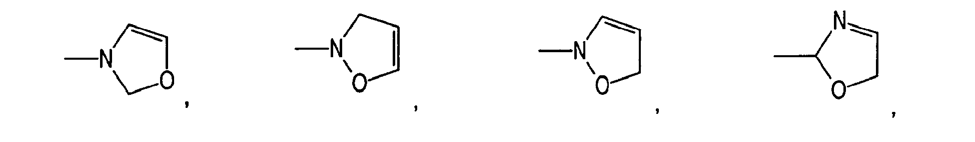 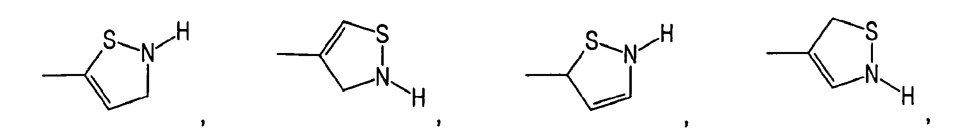 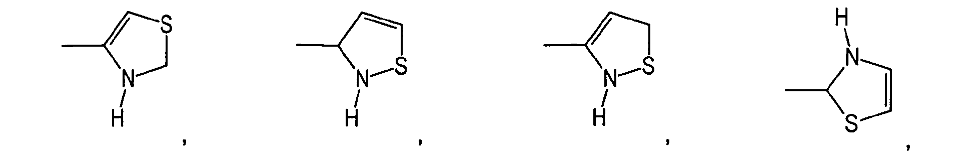 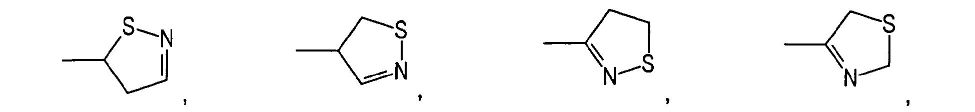 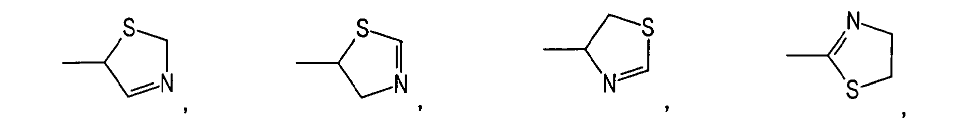 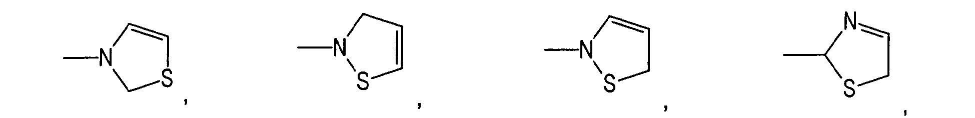 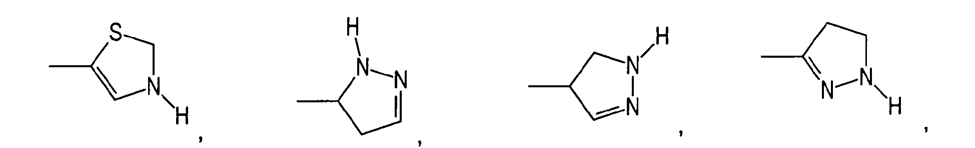 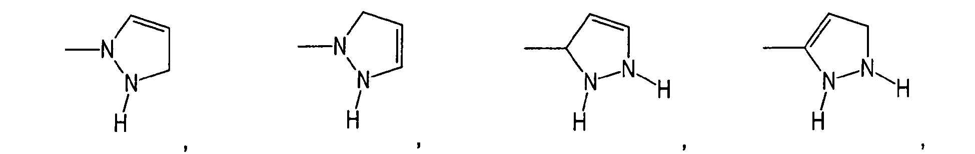 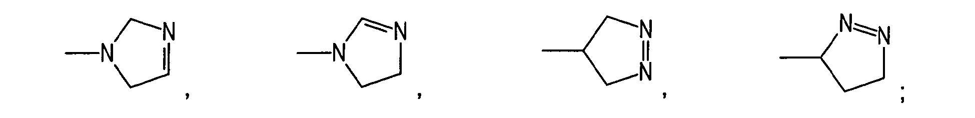
**Z** represents -(CH₂)ₒ- , -O-, -S-, -NH-, -CH=CH-, -C≡C-, -O-(CH₂)ᵣ-, -NH-(CH₂)ₛ-, -CO-, -CO-O-, -NH-CO-, -CO-NH-;
   wherein o, r and s are independently from each other integers from 1 - 6;
**R¹⁷- R⁴⁴** can be selected independently from -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON (CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -N H-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -NH-C(=NH)-NH-cyclo-C₃H₅, -NH-C(=NH)-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -NH-C(=NH)-N[CH(CH₃)₂]₂, -NH-C(=NH)=N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)_{3]2}, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃; -CH₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH₂OH, -CH₂-CH(Cl)-CH₃, -CH₂-CH(Br)-CH₃, -CH₂-CH(I)-CH₃, -CH₂-CH(OH)-CH₃, -CH₂-CH(NH₂)-CH₃, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁,-CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃,-CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, (CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃,-CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C=CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, -CH=CH-Ph, -Ph, -CH₂-Ph;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

The expression prodrug is defined as a pharmacological substance, a drug, which is administered in an inactive or significantly less active form. Once administered, the prodrug is metabolized in the body in vivo into the active compound.

The expression tautomer is defined as an organic compound that is interconvertible by a chemical reaction called tautomerization. Tautomerization can be catalyzed preferably by bases or acids or other suitable compounds.

Preferred are the compounds wherein **X** represents =N-O-(CO)-R¹⁶ and R¹⁶ has the meaning as defined above.

The following compounds [1] - [28] are preferred:

| | | | |
|---|---|---|---|
| 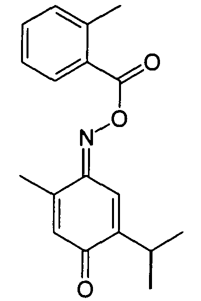 | 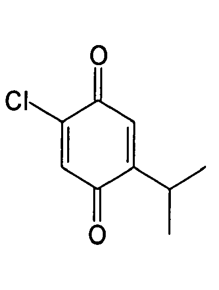 | 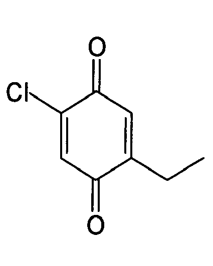 | 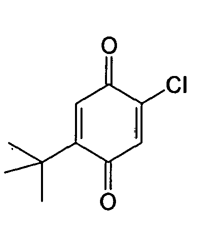 |
| [1] | [2] | [3] | [4] |
| 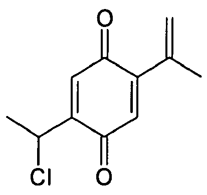 | 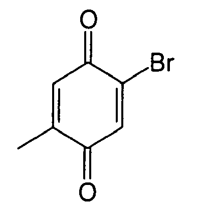 | 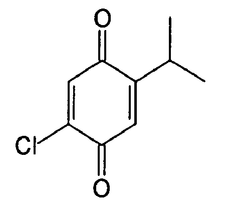 | 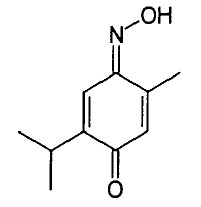 |
| [5] | [6] | [7] | [8] |
| 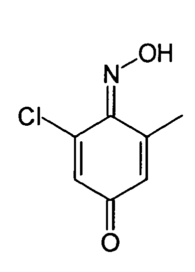 | 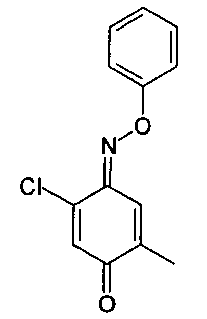 | 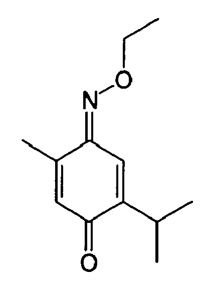 | 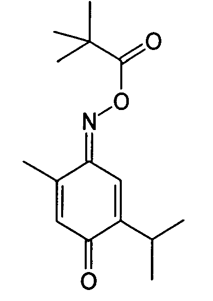 |
| [9] | [10] | [11] | [12] |
| 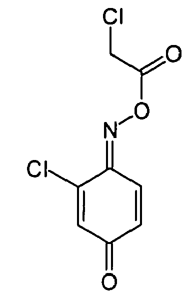 | 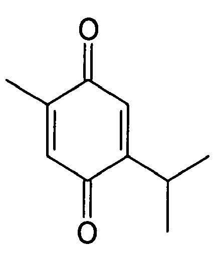 | 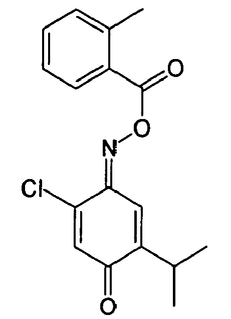 | 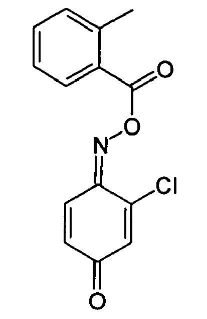 |
| [13] | [14] | [15] | [16] |
| 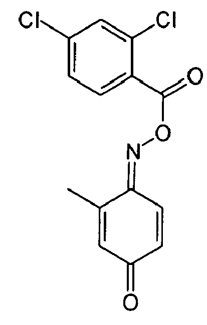 | 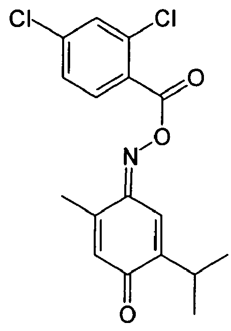 | 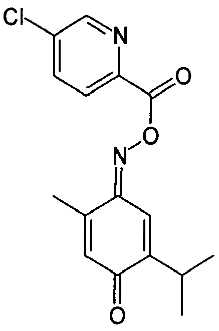 | 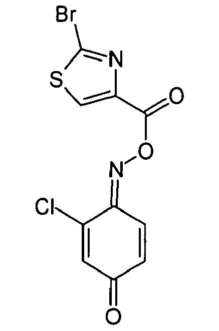 |
| [17] | [18] | [19] | [20] |
| 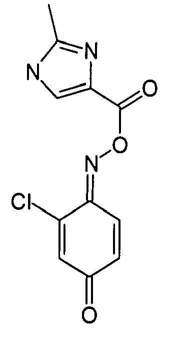 | 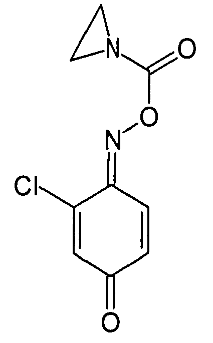 | 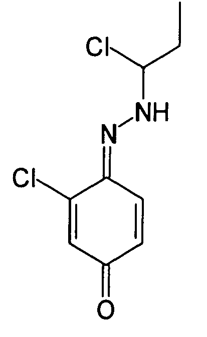 | 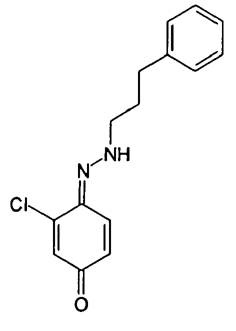 |
| [21] | [22] | [23] | [24] |
| 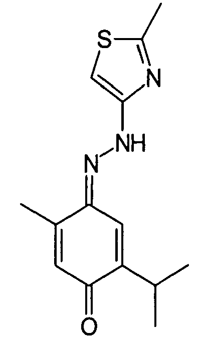 | 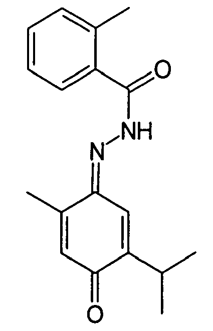 | 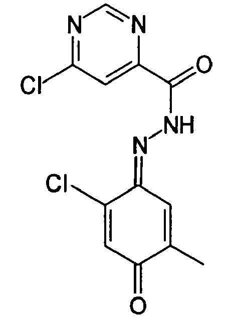 | 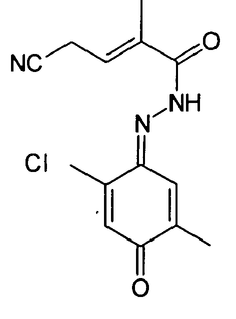 |
| [25] | [26] | [27] | [28] |

Also preferred is when **R¹** to **R⁴** represent independently from each other -H, -F, -Cl, -Br, -CH₃, -CH₂F, -CF₃, -CH₂Cl, -CH₂Br, -CH₂-CH₂F, -CH₂-CF₃, -CH₂-CH₂Cl, -CH₂-CH₂Br, -CH₂-CH₂OH, -CH₂-CH(OH)-CH₃, -CH₂-CH(NH₂)-CH₃, cyclo-C₃H₅, cyclo-C₄H₇, cyclo-C₅H₉, cyclo-C₆H₁₁, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃,-CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH(CH₃)-CH=CH, -CH=C(CH₃)₂,

The preferred substitution pattern of the compounds of the present invention in moiety X is an oxygen or a nitrogen. In case of quinone derivatives (X = O) the chinoid ring (positions R¹ - R⁴) is substituted preferably with alkyl residues and/or halogens as shown in the preferred examples [2] - [7] and [14].

In case of X = NOR the chinoid ring may be substituted as described above. The oxime is may be unsubstituted or bears aliphatic or aromatic residues which may be substituted as shown in the preferred examples [8] - [11].

In case of X = N-O-C=OR the chinoid ring may be substituted as described above and the O-C=O-function can be substituted in R with aliphatic or aromatic moieties which may be further substituted as shown in the preferred examples and [1], [12], [13] and [15] - [22] .

In case of X = NNHR the chinoid ring may be substituted as described above and the hydrazone function can be substituted in R with aliphatic or aromatic moieties which may be further substituted as shown in the preferred list of substances by the examples [23] - [25].

In case of X = N-NH-C=OR the chinoid ring may be substituted as described above and the N-NH-C=OR-function can be substituted in R with aliphatic or aromatic moieties which may be further substituted as shown in the preferred list of substances [26] - [28].

Preferred further substituents in residues R¹ - R⁴ of structure formula (I) are branched or unbranched aliphatic residues which may be further substituted, -NH₂, -NO₂, -CF₃, -Br, -I, -F, -Cl, -CN or hydrogens.

The present invention also comprises pharmaceutically acceptable salts of the compounds according to the general formula (I), all stereoisomeric forms of the compounds according to the general formula (I) as well as solvates, especially hydrates or prodrugs thereof.

In case, the inventive compounds bear basic and/or acidic substituents (the compounds are definitely basic; in addition the compounds may bear acidic substituents), they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Certain compounds of the general formula (I) may exist in the form of optical isomers if substituents with at least one asymmetric center are present, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a (Z)- or (E)-isomer or a mixture thereof. When an isomeric form of a compound of the invention is provided substantially free of other isomers, it will preferably contain less than 5% w/w, more preferably less than 2% w/w and especially less than 1 % w/w of the other isomers.

Another aspect of the present invention relates to the use of the inventive 1,4-benzoquinone and the 1,4-benzoquinone imine derivatives as drugs, i.e. as pharmaceutically active agents applicable in medicine.

Surprisingly it was found that the above-mentioned 1,4-benzoquinone and 1,4-benzoquinone imine derivatives as well as the pharmaceutical compositions comprising said 1,4-benzoquinone and 1,4-benzoquinone imine derivatives are useful for treatment or prophylaxis of cancer and treatment or prophylaxis of inflammations and inflammatory diseases.

Thus, the 1,4-benzoquinone and the 1,4-benzoquinone imine compounds of the present invention can be used for prophylaxis and treatment of cancer or for the preparation of a pharmaceutical formulation for prophylaxis and treatment of cancer and for prophylaxis and treatment of inflammatory diseases.

Examples for cancer types which can be treated and/or prevented by the inventive compounds are selected from the group adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioma, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

Examples for inflammatory diseases which can be treated and/or prevented by the inventive compounds are selected from the group algal disorders, protothecosis, bacterial disorders, abscessation, bacterial meningitis, idiopathic inflammatory disorders, eosinophilic meningoencephalitis, feline polioencephalomyelitis, granulomatous meningoencephalomyelitis, meningitis, steroid responsive meningitis-arteritis, miscellaneous meningitis / meningoencephalitis, meningoencephalitis in greyhounds, necrotizing encephalitis, pug dog encephalitis, pyogranulomatous meningoencephalomyelitis, shaker dog disease, mycotic diseases of the CNS, parasitic encephalomyelitis, prion protein induced diseases, feline spongiform encephalopathy, protozoal encephalitis-encephalomyelitis, toxoplasmosis, neosporosis, sarcocystosis, encephalitozoonosis, trypanosomiasis, acanthamebiasis, babesiosis, leishmaniasis, rickettsial disorders, rocky mountain spotted fever, canine ehrlichiosis, salmon poisoning, viral disorders, aujeszky's disease, borna disease, canine herpes virus encephalomyelitis, canine distemper encephalomyelitis, canine distemper encephalomyelitis in immature animals, multifocal distemper encephalomyelitis in mature animals, old dog encephalitis, chronic relapsing encephalomyelitis, post-vaccinal canine distemper encephalitis, feline immunodeficiency virus, feline infectious peritonitis, feline leukemia virus, infectious canine hepatitis, La Crosse virus encephalitis, parvovirus encephalitis, rabies, post-vaccinal rabies, tick-borne encephalitis in dogs, rheumatoid arthritis, scleroderma, lupus, polymyositis, dermatomyositis, psoriatic arthritis, ankylosing spondylitis, Reiters's syndrome, juvenile rheumatoid arthritis, bursitis, tendinitis (tendonitis), fibromyositis, vasculitis, autoantibodies in vasculitis, microscopic polyangiitis, giant cell arteritis, Takayasu's arteritis, vasculitis of the central nervous system, thromboangiitis obliterans (Buerger's Disease), vasculitis secondary to bacterial, fungal, and parasitic infection, vasculitis and rheumatoid arthritis, vasculitis in systemic lupus erythematosus, vasculitis in the idiopathic inflammatory myopathies, relapsing polychondritis, systemic vasculitis in sarcoidosis, vasculitis and malignancy, drug-induced vasculitis, acute suppurative otitis media, bullous myringitis, granular myringitis, and chronic suppurative otitis media, which can manifest as mucosal disease, cholesteatoma, dermatoses, urticaria (hives), spongiotic dermatitis, allergic contact dermatitis, irritant contact dermatitis, atopic dermatitis, erythemal multiforme (EM minor), Stevens-Johnson syndrome (SJS, EM major), toxic epidermal necrolysis (TEN), chronic inflammatory dermatoses, psoriasis, lichen planus, discoid lupus erythematosus, acne vulgaris, ankylosing spondylitis, Behcet's disease, drug or hypersensitivity reaction, interstitial nephritis, juvenile rheumatoid arthritis, Kawasaki's disease, multiple sclerosis, psoriatic arthritis, Reiter's syndrome, relapsing polychondritis, sarcoidosis, Sjogren's syndrome, systemic lupus erythematosus, ulcerative colitis, vasculitis, vitiligo, Vogt Koyanagi Harada syndrome, acute multifocal placoid pigmentary epitheliopathy, acute retinal necrosis, birdshot choroidopathy, Fuch's heterochromic cyclitis, glaucomatocyclitic crisis, lens-induced uveitis, multifocal choroiditis, pars planitis, serpiginous choroiditis, sympathetic ophthalmia, trauma, gastroesophageal (laryngopharyngeal) reflux disease, pediatric laryngitis, acute laryngeal infections of adults, chronic (granulomatous) diseases, diseases connected with overexpression /overproduction of the protein amyloid A, such as arthritides, rheumatoid arthritis, asthma, bleeding disorders (thrombocytopenia), chronic inflammatory lung diseases, atherosclerosis, kidney inflammation (nephritis), psoriasis, allergies, Crohn's disease, ischemia / reperfusion injury, endotoxemic liver injury, inflammatory bowel disease, tuberculosis, chronic infections, familial Mediterranean fever, interstitial cystitis and skin sunburn, inflammatory diseases caused by viruses like herpes viruses especially cytomegalovirus (causes human coronary heart disease), inflammatory diseases caused by bacteria like mycoplasma pulmonis (causes e.g. chronic lung diseases (CLD), murine chronic respiratory disease), ureaplasma urealyticum (causes pneumonia in newborns), mycoplasma pneumoniae and chlamydia pneumoniae (cause chronic asthma), C. pneumoniae (causes atherosclerosis, pharyngitis to pneumonia with empyema, human coronary heart disease), helicobacter pylori (human coronary heart disease, stomach ulcers), inflammatory diseases caused by prions, inflammaotory diseases caused by fungi like histoplasmosis, coccidioidomycosis, candidiasis, aspergillosis, sporotrichosis, blastomycosis, inflammaotory diseases caused by spirochetes or bacteria like tuberculosis, leprosy, proprionobacterium, syphilis, Whipple's disease, leptospirosis, brucellosis, and lyme disease, inflammaotory diseases caused by parasites (protozoan or helminthic) like toxoplasmosis, acanthameba, toxocariasis, cysticercosis, onchocerciasis and allergic, immune, and idiopathic disorders and miscellaneous inflammatory conditions.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders, deposits and microparticles as well as nanoparticles.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzothiophene-1,1-dioxide derived compound and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The compounds according to the present invention may also be bound to nanoparticles or may form particles in the size of micrometers to nanometers. The term nanoparticles as recited herein refers to particles which have a diameter smaller than 1 µm. The nanoparticles can be coated with one or more layers in which one or more compounds according to the present invention can be seeded. The compounds according to the present invention may also be bound with linker groups to the nanoparticle or maybe bound directly to the nanoparticle core.

Preferably are nanoparticles consist of a magnetic more preferably superparamagnetic core such as maghemite or magnetite which can be coated with biological compounds such as polymers and antibodies which increase the affinity of the nanoparticles to tumorous tissue and cancer cells. The inventive compounds can be bond adhesively or covalently to such nanoparticles or can simply be administered together with the nanoparticles. A preferred route of administration is direct infusion into the cancerous tissue. Consequently, the treatment of solid tumours is most preferred.

Moreover such nanoparticles or microparticles are most preferred which can be directed by the use of an magnetic field to the treatment side and/or which can be activated by an alternating magnetic field which may warm up said nanoparticles or microparticles due to hysteresis heat. Such generation of heat by said particles due to the applications of an alternating magnetic field supports cancer treatment since cancer cells are more sensible to heat than healthy cells.

Consequently, a preferred pharmaceutical formulation contains magnetic preferably superparamagnetic nanoparticles together with at least one inventive compound.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions may further comprise at least one active 1,4-benzoquinone or 1,4-benzoquinone imine of the general formula (I).

The pharmaceutical compositions may further comprise at least one drug selected from the group comprising meclofenoxate, nicergoline, piracetame, pyritinole, tacrine, donezepiel, galantamine, rivastigmine, memantine, IMPase-inhibitors and extracts from Gingko biloba.

### General procedure for the preparation of 1,4-benzoquinone and 1,4benzoquinone imine derivatives

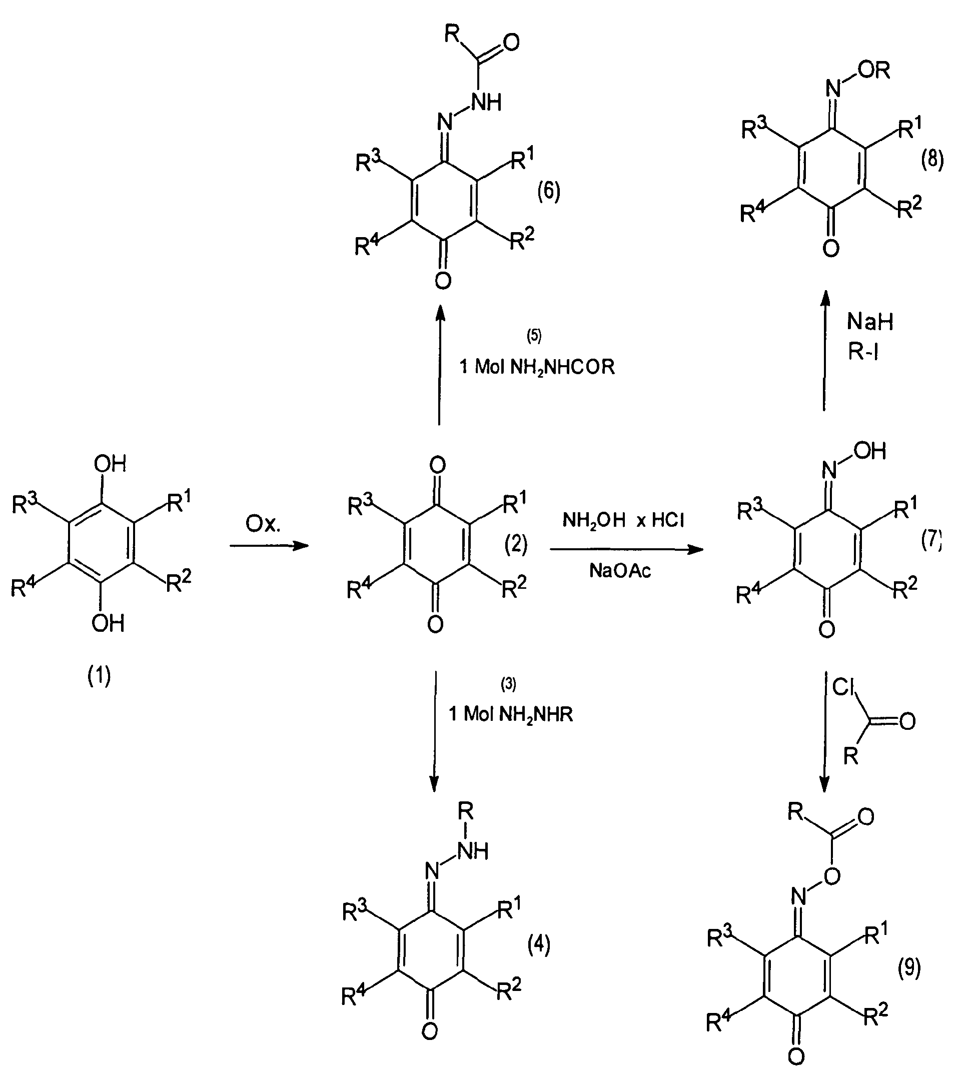

The hydroquinone (1) was dissolved in an appropriate solvent at room temperature and oxidized according to known methods resulting in 1,4-benzoquinone derivative (2). To form hydrazone (4) benzoquinone (2) was reacted with 1 mole of reagent (3) under standard conditions. To form derivative (6) benzoquinone (2) was reacted with compound (5) under standard conditions. To achieve oxime (7) hydroxylammonium chloride was added to a stirred mixture of (2) in presence of sodium acetate. The resulting oxime (7) can be further reacted with a iodo-alkyl compound in presence of sodium hydride to achieve O-alkylated oxime (8) or can be reacted with an appropriate acid chloride to form compound class (9) according to standard procedures.

### Description of the figures:

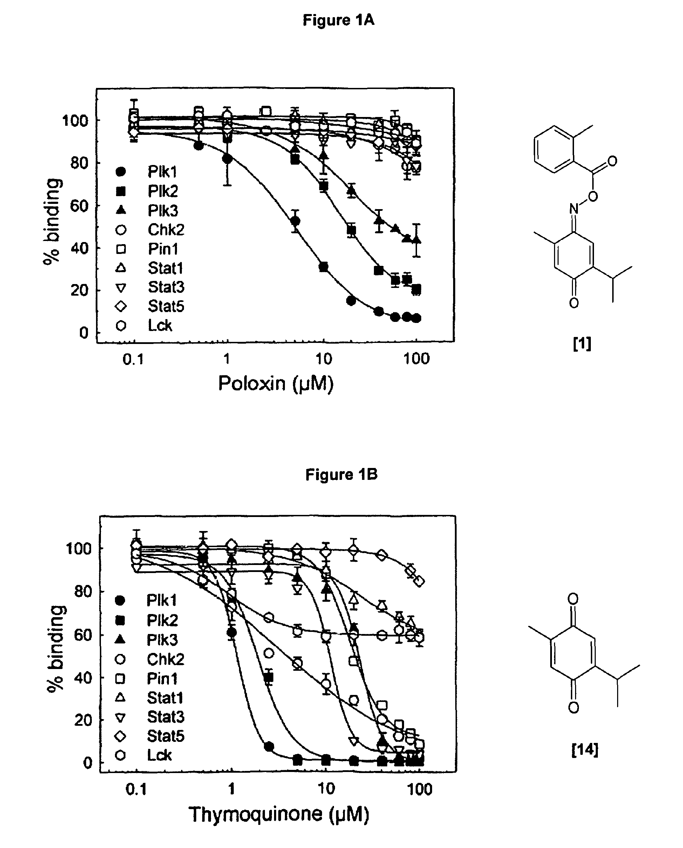

- Figure 1A/B: Comparative IC₅₀ inhibition curves with preferred compounds [1] (poloxin) and [14] (thymoquinone, TQ) versus Plk1, Plk2, Plk3, Chk2, Pin1, Stat1, Stat3, Stat5 and Lck.

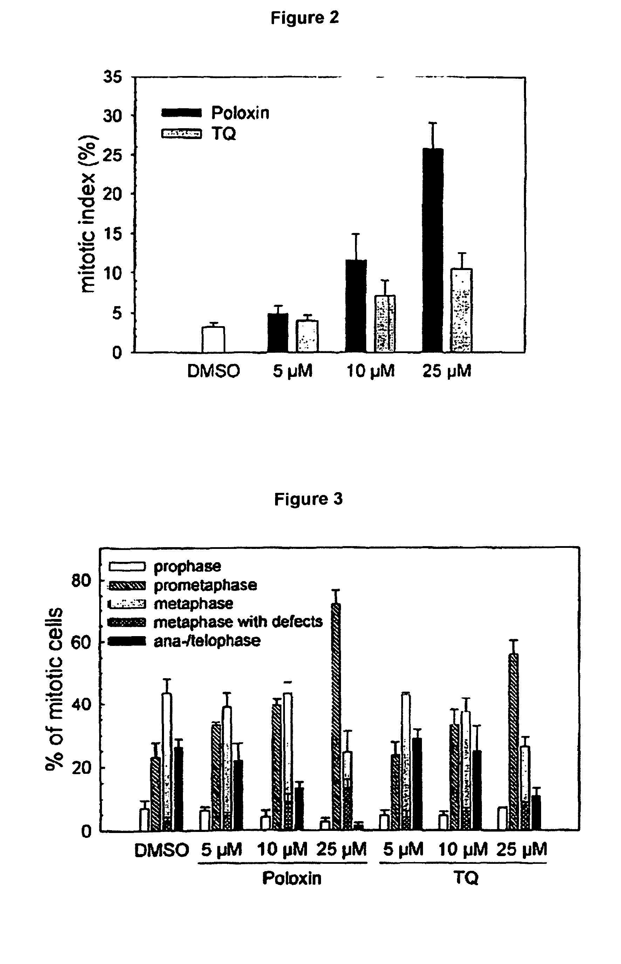

- Figure 2: Cellular effects of poloxin and thymoquinone (TQ): Mitotic indices of HeLa cells 14 hrs after release from G1/S block.
- Figure 3: Distribution of mitotic phases and chromosome congression defects within the metaphase population in the presence of poloxin and thymoquinone (TQ).
- Figure 4: Quantitative analysis of the effects of poloxin (n= 20) and thymoquinone (TQ) (n=16) on centrosomal Plk 1 localization in metaphase cells compared to DMSO (n=20).
- Figure 5A: Poloxin and Thymoquinone induce mitotic arrest in HeLa cells (Figs. 5A-5C):
HeLa cells were synchronized by thymidine block and released into medium containing Poloxin or Thymoquinone (TQ) at the indicated concentrations as described below. Unsynchronized cells (control), cells synchronized with nocodazol (Noc) or thymidine (Thym), or cells released from thymidine block in the medium containing only DMSO (DMSO) were analyzed for comparison.

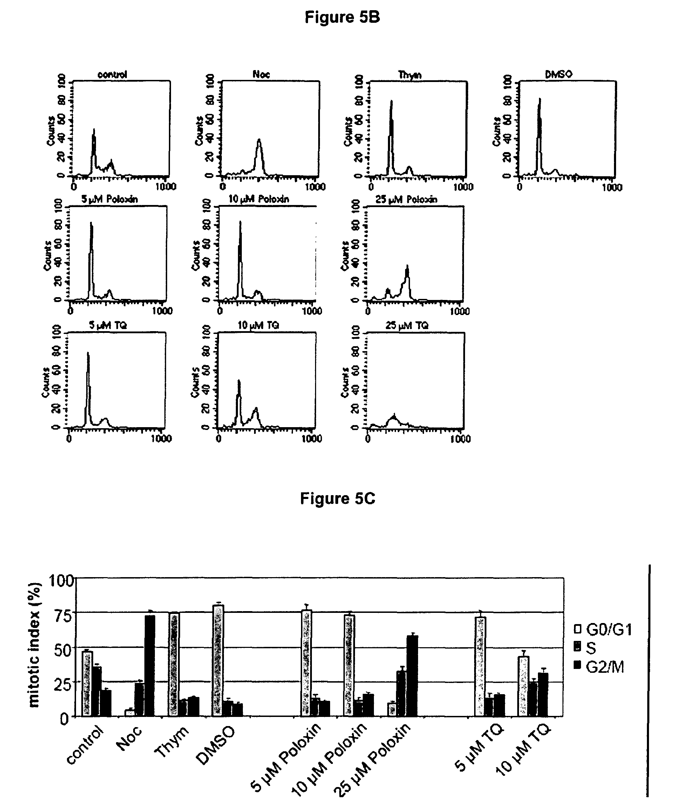

- Figure 5B: Analysis of cell cycle distribution by fluorescence-activated flow cytometry. Cells were treated as in 5A. A representative example is shown.
- Figure 5C: Quantification of the cell cycle analysis as shown in 5B (n=3). Quantification of the cell cycle profile in the presence of 25 µM Thymoquinone (TQ) was unfeasible due to toxic side effects.

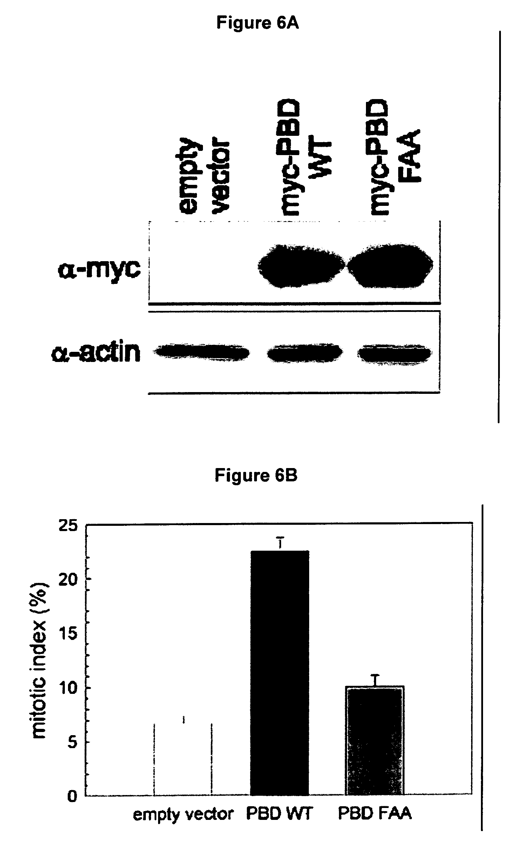

- Figure 6A: Mislocalisation of endogenous Plk1 by overexpression of the PBD induces mitotic arrest (Figs. 6A + 6B): Overexpression of myc-tagged PBD wild-type (wt) and an inactive triple mutant (PBD FAA) in HeLa cells.
- Figure 6B: Overexpression of PBD wild-type (wt), but not of the triple mutant PBD FAA, induces mitotic arrest in HeLa cells.

### Examples

### Example 1: Plasmid construction and protein expression

DNA sequences coding for human Plk1 amino acids 326-603, human Plk2 amino acids 355-685, and human Plk3 amino acids 335-646 comprising the respective polo-box domains were amplified by PCR from plasmid DNA (Plk1) or placenta cDNA (Plk2, Plk3) and cloned into a modified pET28a (Plk1) or a modified pQE70 vector (Plk2 and Plk3). Similarly, nucleotides coding for the forkhead homology domain (FHA) of the kinase Chk2 (amino acids 1-225) and the WW domain of the proline isomerase Pin1 (amino acids 1-162) were amplified by PCR from HeLa S3 and placenta cDNA, respectively, and cloned into a modified pET28a vector. The design of expression plasmids coding for STAT1, STAT3, STATSb, and Lck has been described before and belongs to the knowledge of a skilled person. Site-directed mutagenesis of the Plk1 PBD was applied to generate the inactive Plk1 PBD mutant (PBD FAA) displaying three mutations: W414F, H538A, and K540A. Wild-type and mutant PBD sequences were cloned into a modified pCS2 mammalian expression vector carrying a myc-tag.

Proteins were expressed from Rosetta BL21 DE3 (Novagen), purified by affinity chromatography, and dialyzed against a buffer containing 50 mM Tris (pH 8.0), 200 mM NaCl (for Plk1: 400 mM NaCl), 1 mM EDTA, 1 mM dithiothreitol, 10% glycerol and 0.1 % Nonidet P-40. The expression and purification for STAT1, STAT3, STAT5b, and Lck has been described before and belongs to the knowledge of a skilled person. Purified proteins were aliquoted, snap-frozen in liquid nitrogen, and stored at -80°C before use. Binding curve experiments were done for each protein batch before inhibition experiments were started.

### Example 2: Transient transfection of cells and cellular extract preparation

Plasmid transfections were carried out using the FuGENE6 reagent (Roche) according to the manufacturer's instructions. 48 hours after transfection cells were harvested and lysed in 50 mM Hepes (pH 7.5), 150 mM NaCl, 1 mM EDTA, 10% glycerol, 1% Triton X-100, 10 mM Na₂P₂O7, 10 mM NaF, 2 mM Na₃VO₄, 1 mM Aprotinin, 0.5 mM PMSF. Protein levels were analyzed by Western blot analysis. Proteins were probed with a mouse monoclonal anti-myc antibody (1:5000, Santa Cruz) and a rabbit polyclonal anti-actin antibody (1:5000, Sigma-Aldrich), followed by incubation with a goat polyclonal anti-mouse or a swine polyclonal anti-rabbit horseradish peroxidase (HRP)-conjugated secondary antibody (both 1:7500, Dako). Cells transiently transfected in parallel were used for immunofluorescence assays.

### Example 3: High-throughput screening and fluorescence polarization assays

Diverse Chemical libraries consisting of 22461 small molecules were tested in a fluorescence polarization assay which analyzes binding between 5-carboxyfluorescein-GPMQST(P0₃H2)PLNG-OH (final concentration: 10 nM) and the Plk1 polo-box domain (final concentration: 65 nM). The final concentration of buffer components used was: 10 mM Tris, pH 8.0, 50 mM NaCl, 1 mM EDTA, 0.1 % Nonidet P-40, and 10% DMSO. The Plk1 PBD was incubated with compounds for 60 min prior to addition of the fluoropeptide. Screening was performed at room temperature. The following fluorophore-labeled peptides were used for specificity assays:
Plk2: 5-carboxyfluorescein-GPMQTST(PO₃H₂)PKNG-OH;
Plk3: 5-carboxyfluorescein-GPLATST(PO₃H₂)PKNG-OH;
Chk2: 5-carboxyfluorescein-GHFDT(PO₃H₂)YLIRR-OH;
Pin 1: 5-carboxyfluorescein-GWFYS(PO₃H₂)PRLKK-OH;
STAT1: 5-carboxyfluorescein-GY(PO₃H₂)DKPHVL-OH;
STAT3: 5-carboxyfluorescein-GY(PO₃H₂)LPQTV-NH₂;
STAT5b: 5-carboxyfluorescein-GY(PO₃H₂)L VLDKW-OH;Lck:5-carboxyfluorescein-GY(P0₃H₂)EEIP-OH.

Proteins were incubated at 22 °C with the test compounds for 1 hr prior to addition of the fluorophore-labeled peptides, and were used at the following final concentrations, which correspond approximately to the Kd-values of the respective assays: Plk1: 65 nM, Plk2: 130 nM, Plk3: 1875 nM, Chk2: 240 nM, Pin1: 1000 nM, STAT1: 120 nM, STAT3: 160 nM, STAT5b: 110 nM; Lck: 40 nM. Fluorescein-labeled peptides were >95% pure. Poloxin [1] and thymoquinone [14] were synthesized according to the general procedure mentioned above. Inhibition curves were fitted using SigmaPlot (SPSS Science Software GmbH).

### Results: Activities of Poloxin and Thymoquinone against phosphoserine-, phosphothreonine-, and phosphotyrosine-binding protein domains

Concentrations of inhibitors required for half-maximal inhibition (apparent IC₅₀-values) under the conditions described in Supplementary Methods (incubation of the proteins with the inhibitors for 1 hr at 22 °C prior to addition of fluorophore-labeled peptides) are given.

| | **Poloxin** apparent IC₅₀ | **Thymoquinone** apparent IC₅₀ |
|---|---|---|
| Plk1 PBD | 4.8+/-1.3µM | 1.14+/-0.04µM |
| Plk2 PBD | 18.7+/-1.8µM | 1.90+/-0.10 µM |
| Plk3 PBD | 53.9 +/- 8.5 µM | 22.4 +/- 0.8 µM |
| Chk2 FHA domain | >330µM | 3.9 +/- 0.6 µM |
| Pin1 WW domain | >215 µM | 20.4 +/- 0.9 µM |
| STAT1 SH2 domain | > 555 µM | >5mM |
| STAT3 SH2 domain | > 285 µM | 10.9+/-0.2 µM |
| STAT5 SH2 domain | >1mM | > 240 µM |
| Lck SH2 domain | >5 mM | >5 mM |

### Example 4: Western blot analysis

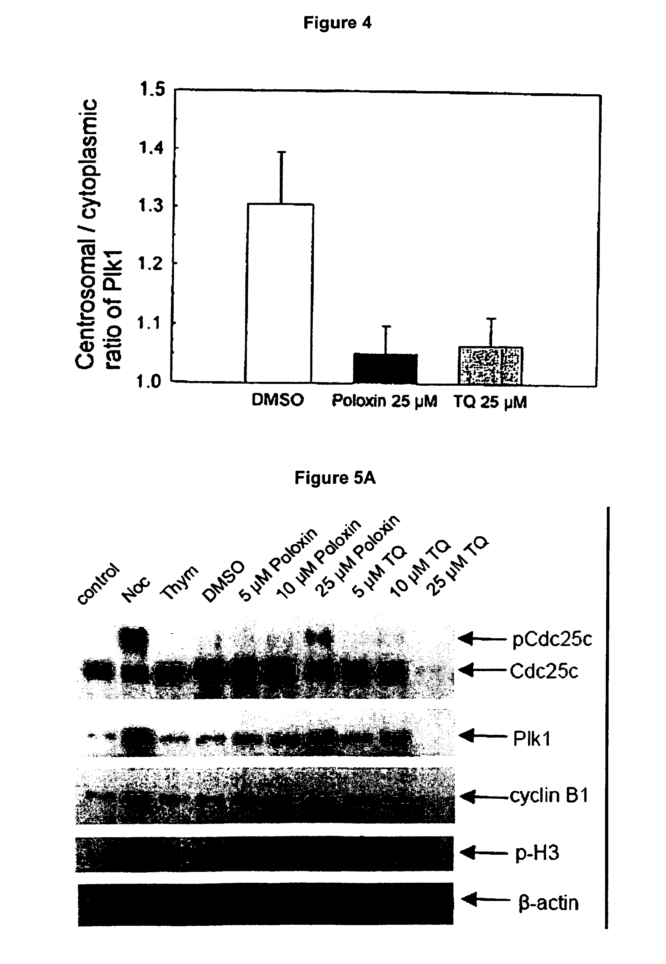

HeLa cells were maintained in Dulbecco's Modified Eagle-Medium (High Glucose) supplemented with 2 mM L-glutamine, 1 mM sodium pyruvate, 10% foetal calf serum, 100 U/mL penicillin, and 100 µg/mL streptomycin (GIBCO and PAA). For the Western blot analysis Fig. 5A, HeLa cells were arrested at the G1/S boundary by thymidine block, and released into thymidine-free medium containing the indicated concentration of compounds at a final DMSO concentration of 0.5%. After 7 hours, medium was replaced with fresh medium which also contained the indicated concentration of compound. 14 hours after the initial release cells were lysed with RIPA buffer (50mM Tris-HCl pH 8.0, 150mM NaCl, 1 % NP-40, 0.5% Na-desoxycholate, 0.1 % SDS, 1 mM Na₃VO₄, 1 mM PMSF, 1 mM DTT, 1 mM NaF, and protease inhibitor Cocktail Complete (Roche)). Western blot analysis was performed according to standard protocol using the corresponding antibodies. Mouse monoclonal antibody against Plk1 (1:250), mouse monoclonal antibody against cyclin B1 (1:15000), rabbit polyclonal antibody against Cdc25C (1:100) and goat anti-mouse or anti-rabbit secondary antibodies (1:4000) were purchased from Santa Cruz. Mouse monoclonal antibody against β-actin (1:100000) was obtained from Sigma-Aldrich. Rabbit polyclonal anti-phospho-histone 3 antibody (1:100) was purchased from Upstate.

### Example 5: Cell cycle analysis

For the determination of mitotic indices shown in Fig. 6A, the number of mitotic cells within a population of 200-300 cells was counted. For the determination of the mitotic phase distribution shown in Fig. 6B, populations of approximately 50 mitotic cells were analyzed. All experiments were performed in triplicate. For the cell cycle analysis shown in Figs. 5B and 5C, cells were harvested, washed with PBS, fixed in chilled 70% ethanol at 4°C for 30 min, treated with 1 mg/ml of RNase A (Sigma-Aldrich) for 30 min and stained with 100 µg/ml of propidium iodide. The DNA content of 30000 cells was determined with a FACScan flow cytometer (BD Biosciences). The data were analyzed with cell cycle analysis Software MODFIT LT 2.0 (Verity Software House). All experiments were performed in triplicate.

### Example 6: Apoptosis assay

Apoptosis was assessed using the Vybrant apoptosis assay kit no.2 according to the manufacturer's instructions (Molecular Probes).

### Example 7: Immunofluorescence Assay

He La cells were grown on coverslips for 24 hours, and arrested in G1/S by addition of 1 µg/ml aphidicolin for 14 hours. Cells were released into aphidicolin-free medium containing the indicated concentration of compounds at a final DMSO concentration of 0.5%. After 7 hours, the medium was replaced with fresh medium which also contained the indicated concentration of compound. 14 hours after the initial release, cells were fixed and permeabilized in methanol for 15 min at -20°C. Cells were washed in PBS and incubated for 60 min in blocking solution (PBS with 0.1 % Tween-20, 1% goat serum). All antibodies were diluted in blocking solution, and incubations were carried out for 1 hour at room temperature in a humidified chamber, followed by 3 washes with PBS containing 0.1 % Tween-20. The following antibodies were used: mouse monoclonal anti-gamma-tubulin (1:1000, Sigma-Aldrich), which was detected with Alexa Fluor 546-conjugated goat anti-mouse (1:1000, Molecular Probes), mouse monoclonal anti-alpha-tubulin-FITC (1:500, Sigma-Aldrich), and rabbit polyclonal anti-Plk1 (1:75, Biozol), which was detected by Alexa Fluor 488-conjugated goat anti-rabbit (1:1,000, Molecular Probes). DNA was stained with 4',6-diamidino-2-phenylindole (DAPI, 2 µg/ml, Sigma-Aldrich). Immunofluorescence microscopy was performed on a DeltaVision Microscope (Applied Precision) at 100x magnification. Images were processed using a deconvolution algorithm of the Softworx Software (Applied Precision). The centrosomal/cytoplasmic ratio of Plk1 staining was determined by measuring the total intensity in a circular region of fixed diameter round the centrosome relative to the intensity in a cytoplasmic region of the same size. For quantification, nondeconvolved images with identical exposure times were used.

## Claims

1. Compounds having the general formula (I): 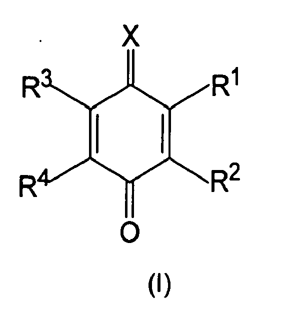 wherein
**R¹ - R⁴** represent independently from each other -H, -R⁵, -R⁶, -R⁷, -R⁸, -CR⁹R¹⁰R¹¹, -Y-CR¹²R¹³R¹⁴, -Y-R¹⁵
**Y** represents independently from each other -(CH₂)ₙ-, -O-, -S-, -NH-, -CH=CH-, -C≡C-, -O-(CH₂)ₘ-, -NH-(CH₂)ₚ-, -CO-, -CO-O-, -NH-CO-, -CO-NH-;
wherein n, m and p are independently from each other integers from 1 - 6;
**R⁵ - R¹⁵** represent independently from each other -H, -F, -Cl, -Br, -I, -CH₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -CH₂-CH₂OH, -CH₂-CH(Cl)-CH₃, -CH₂-CH(Br)-CH₃, -CH₂-CH(I)-CH₃, -CH₂-CH(OH)-CH₃, -CH₂-CH(NH₂)-CH₃, cydo-C₃H₅, cyclo-C₄H₇, cyclo-C₅H₉, cyclo-C₆H₁₁, cyclo-C₇H₁₃, cyclo-C₈H₁₅, -Ph, CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁,-CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃,-CH(C₂H₅)₂, -C₂H₄-CH(CH₃)₂, -C₆H₁₃, -C₇H₁₅, -C₈H₁₇, -C₃H₆-CH(CH₃)₂,-C₂H₄-CH(CH₃)-C₂H₅, -CH(CH₃)-C₄H₉, -CH₂-CH(CH₃)C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂,-C₃H₆-CH=CH₂, -C₂H₄-CH=CH-CH₃, -CH₂-CH=CH-CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH-C₂H₅, -CH=CH-C₃H₇, -CH₂-CH=CH-CH=CH₂, -CH=CH-CH=CH-CH₃, -CH=CH-CH₂-CH=CH₂,-C(CH₃)=CH-CH=CH₂, -CH=C(CH₃)-CH=CH₂, (CH₃)-CH=CH-CH₃, -CH=CH-CH(CH₃)₂, -CH=C(CH₃)-C₂H₅. -C(CH₃)=CH-C₂H₅,-C(CH₃)=C(CH₃)₂, -C(CH₃)₂-CH=CH₂, -CH(CH₃₎-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)_{2,} -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH_{2,} -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH_{2,} -CH=CH-CH₂-C(CH₃)=CH₂, -CH(CH₃)-C≡C-CH₃-CH=CH-CH(CH₃)-CH=CH_{2,} -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃₎-CH₂-C≡CH, -CH=CH-C(CH₃)=CH-CH₃, -CH=C(CH₃)-CH=CH-CH_{3,} -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C=C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH_{3,} -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, -CH=CH-Ph, -Ph, -CH₂-Ph;
**X** represents =O, =N-OR¹⁶, =N-NHR¹⁶, =N-O-(CO)-R¹⁶, =N-NH-(CO)-R¹⁶;
**R¹⁶** represents -R⁴¹, -CR⁴²R⁴³R⁴⁴, -Z-CR⁴²R⁴³R⁴⁴, -Z-R⁴¹. 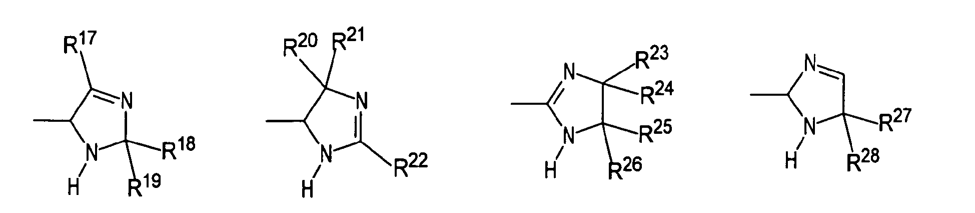 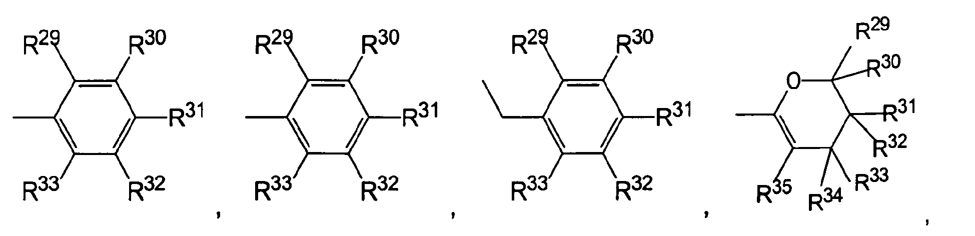 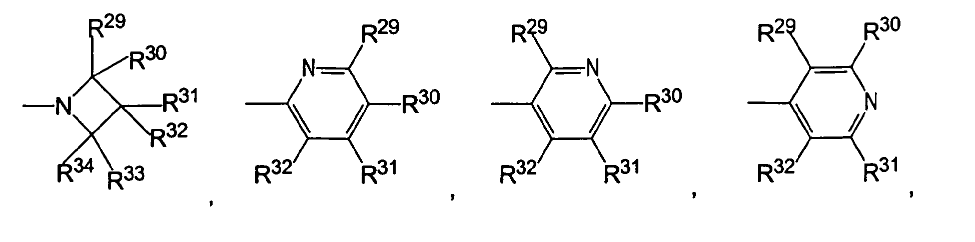 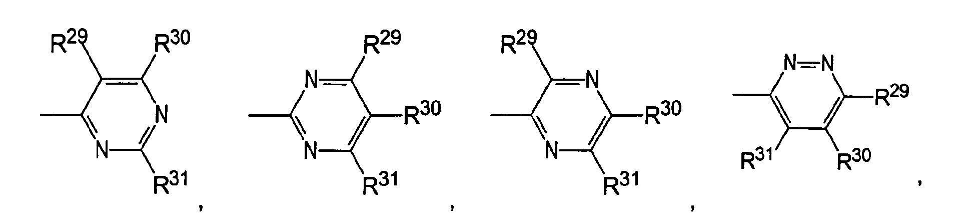 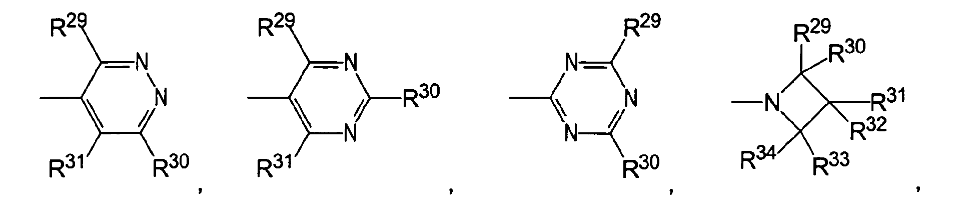 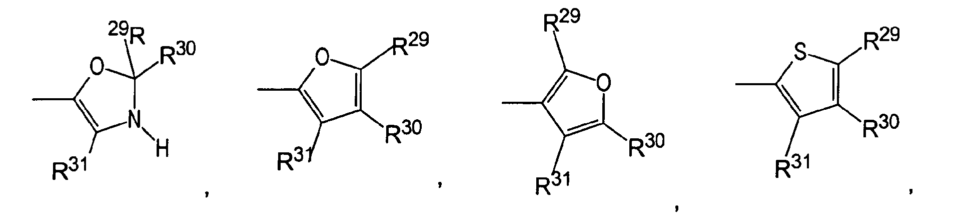 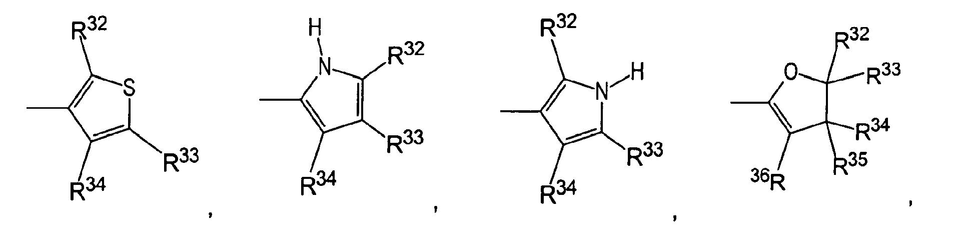 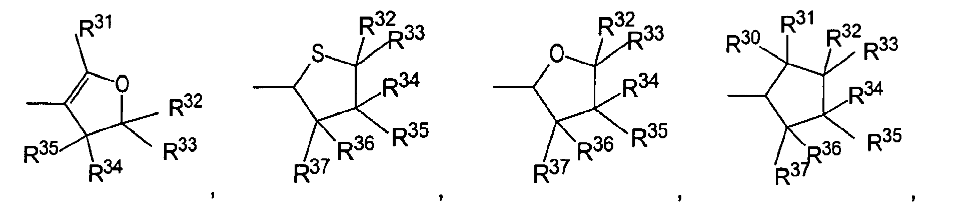 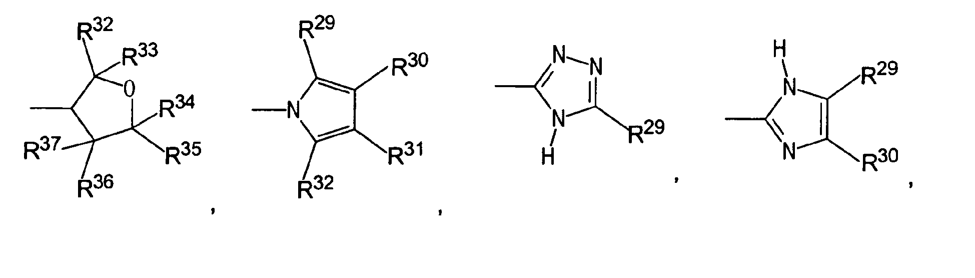 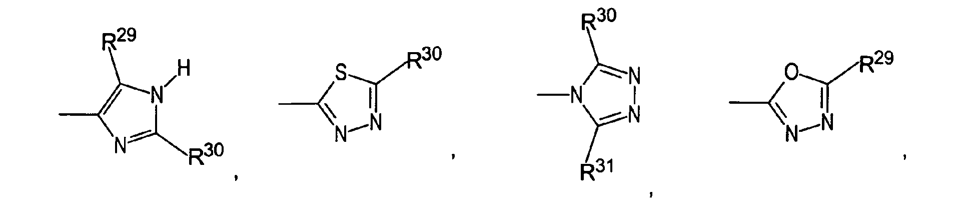 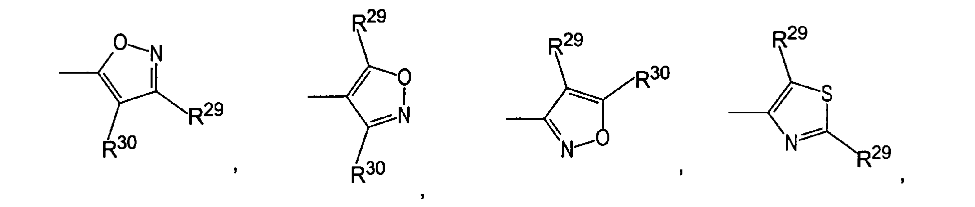 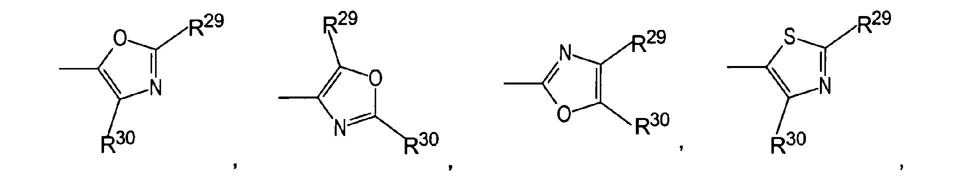 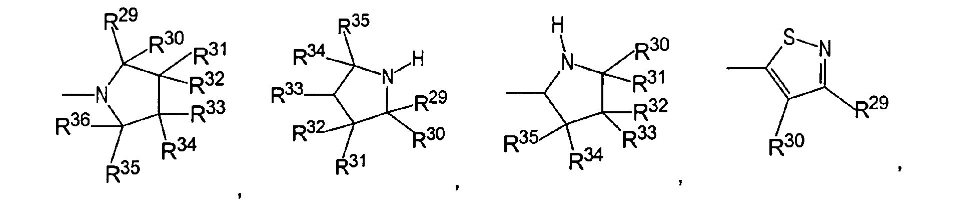 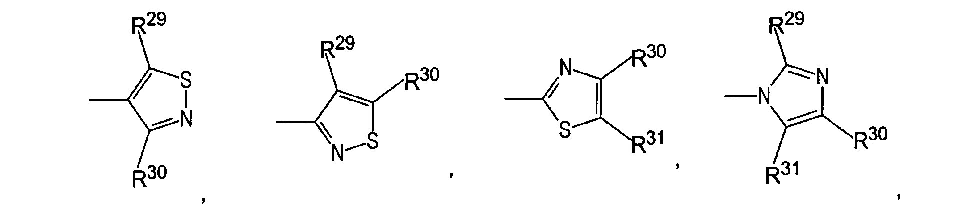 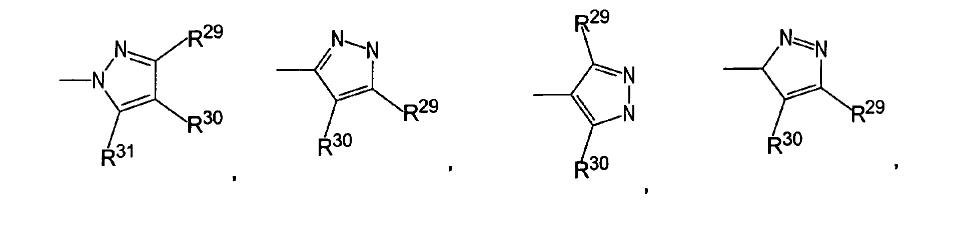 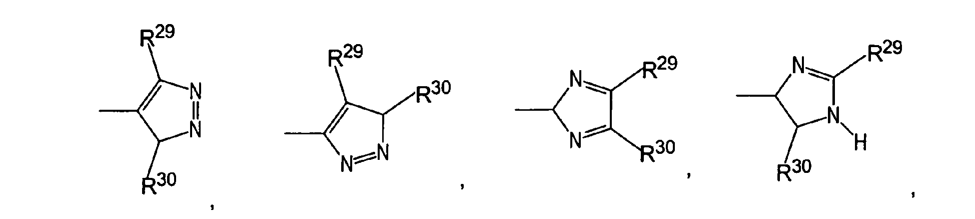 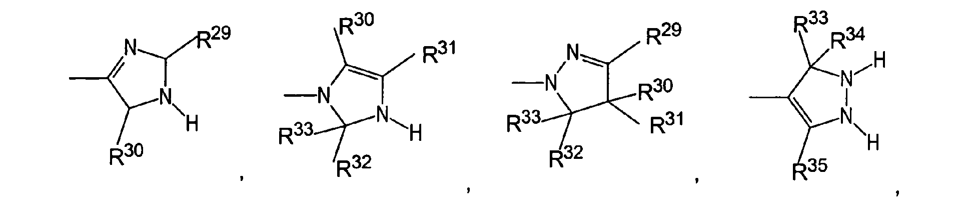 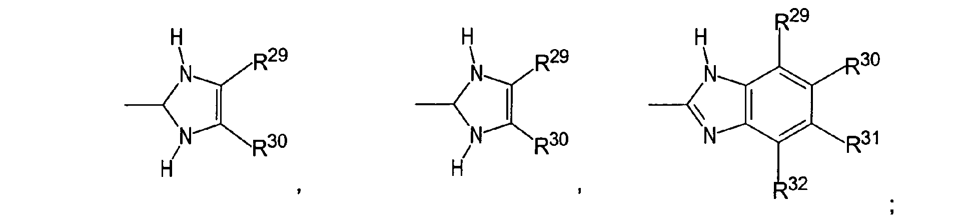 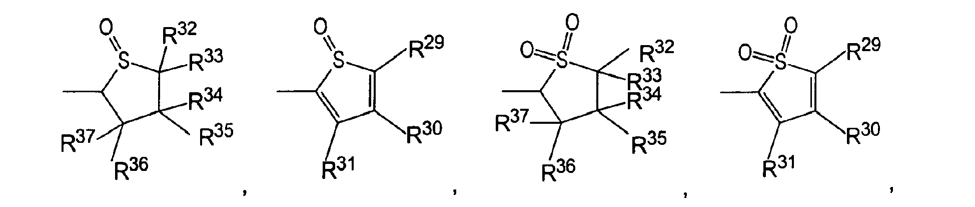 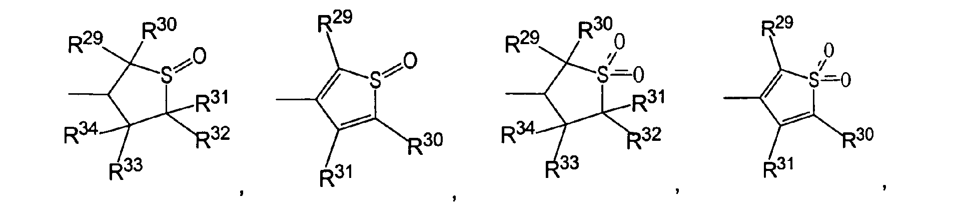 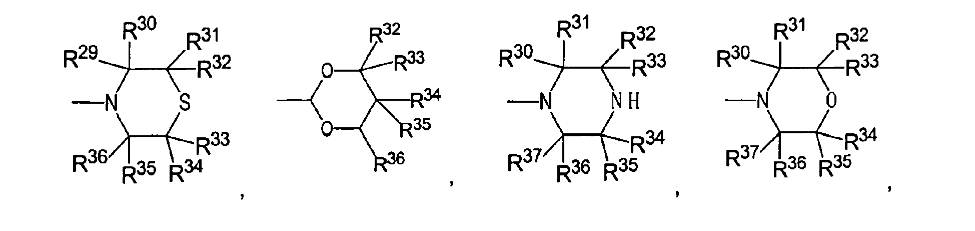 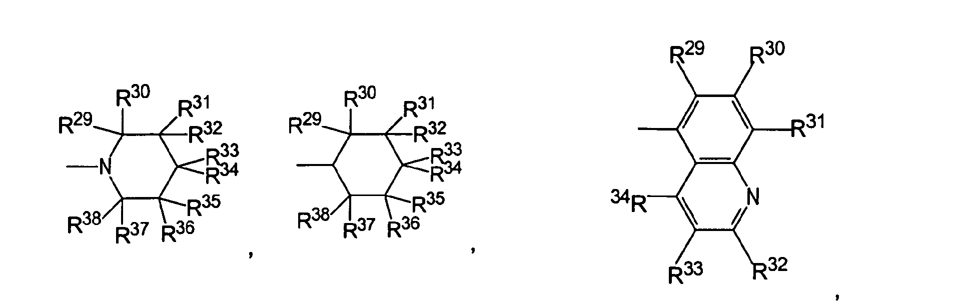 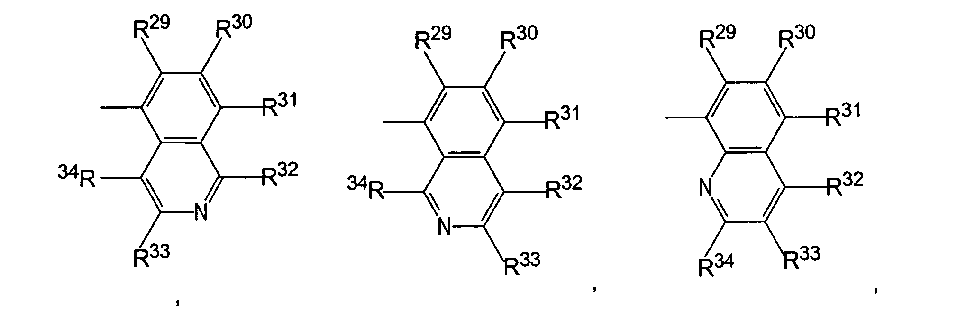 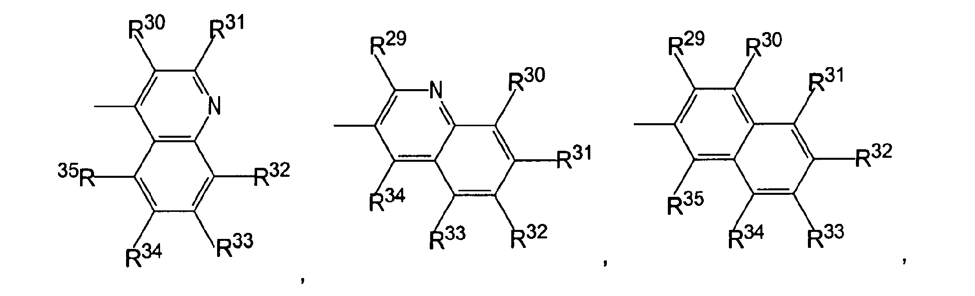 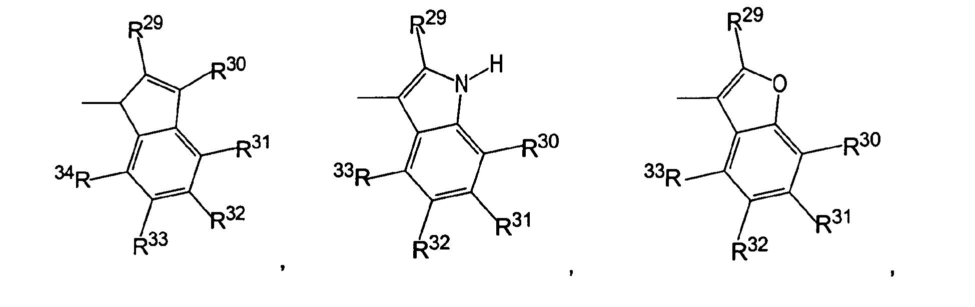 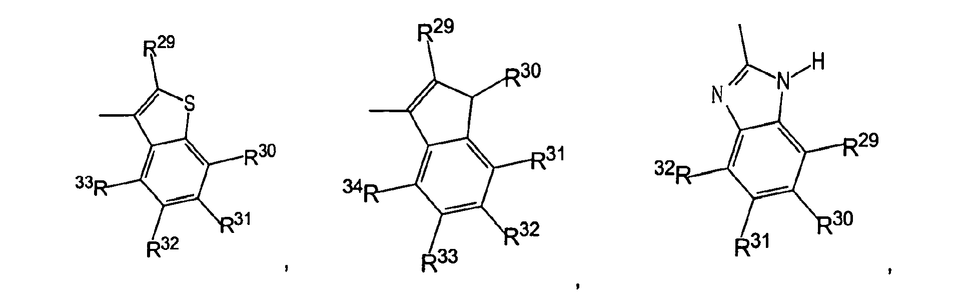 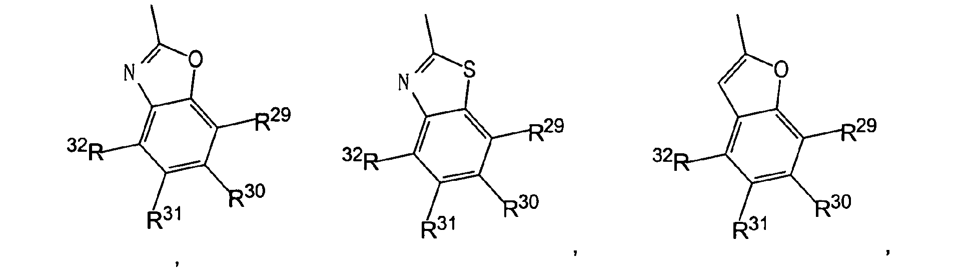 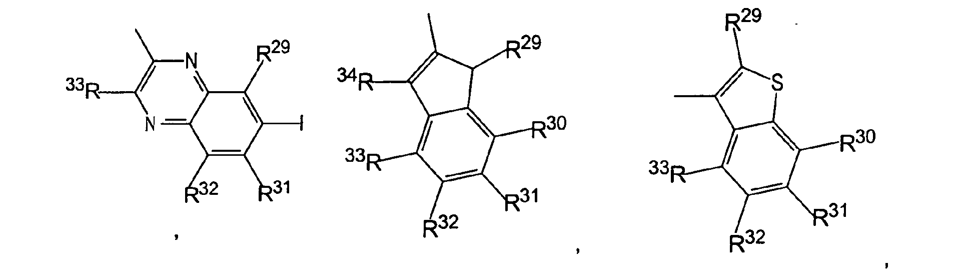 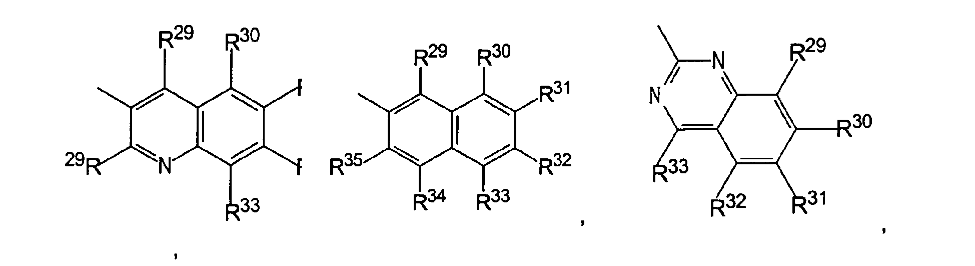 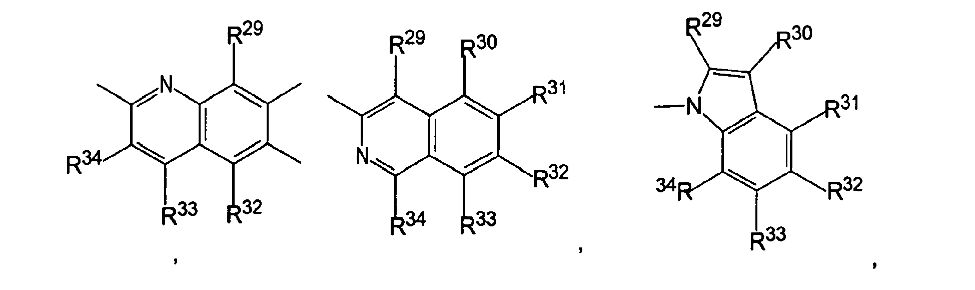 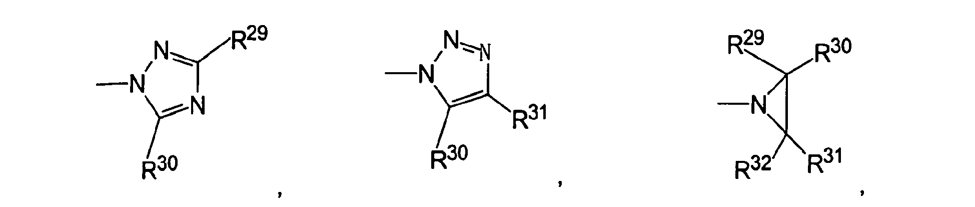 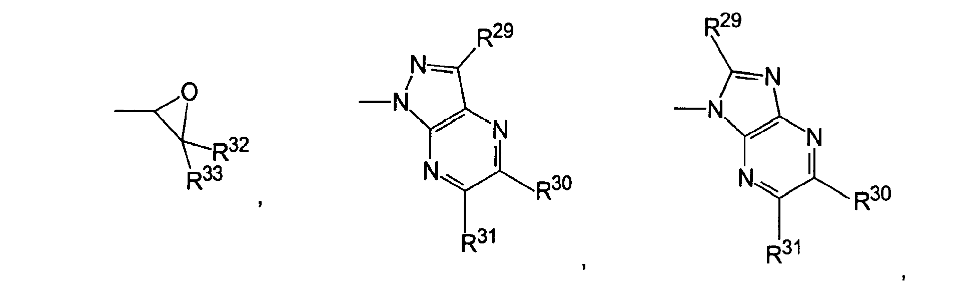 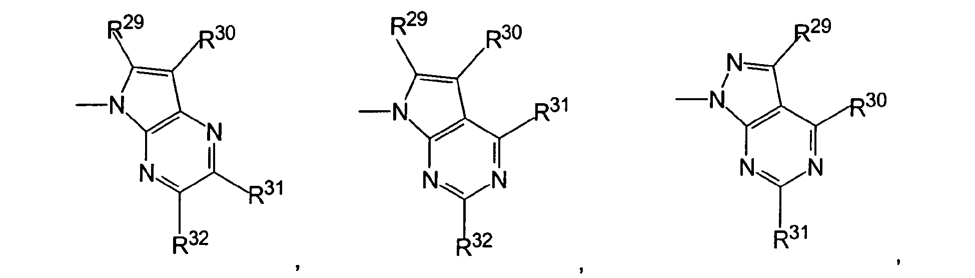 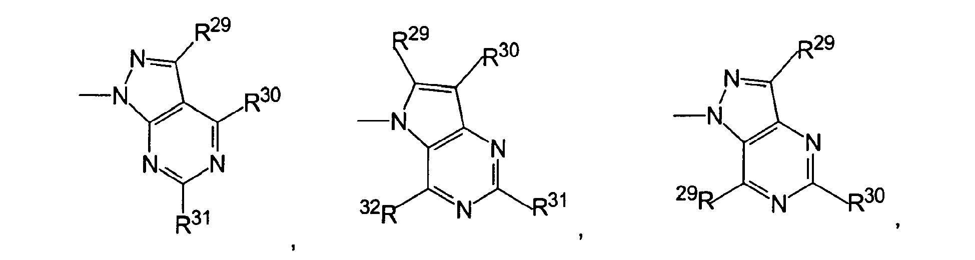 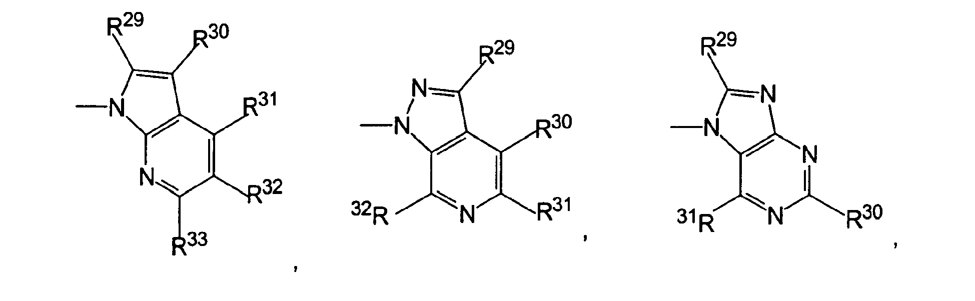 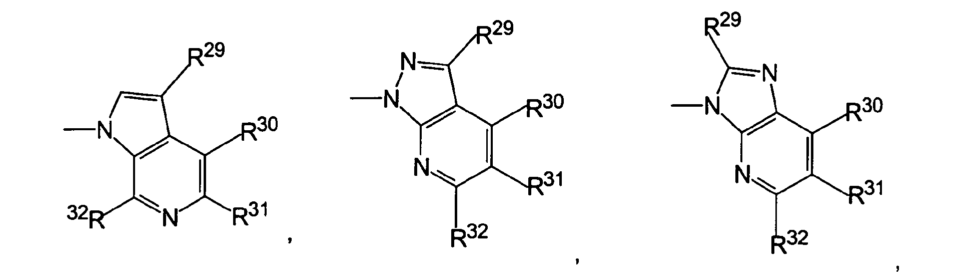 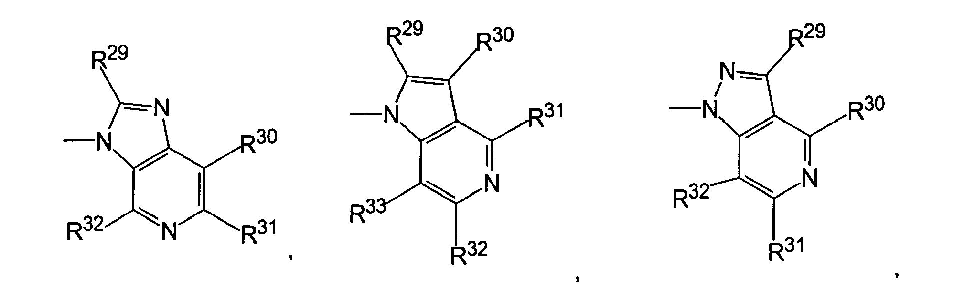 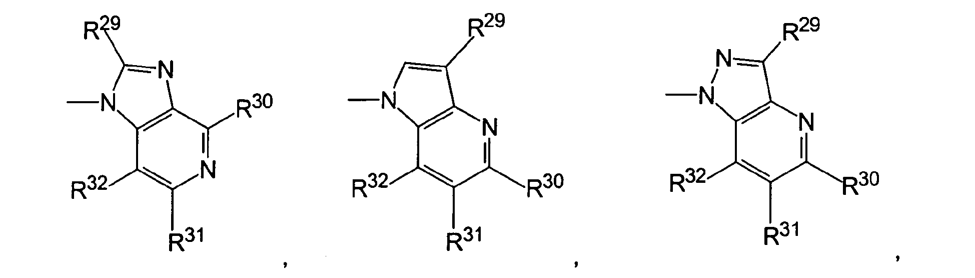 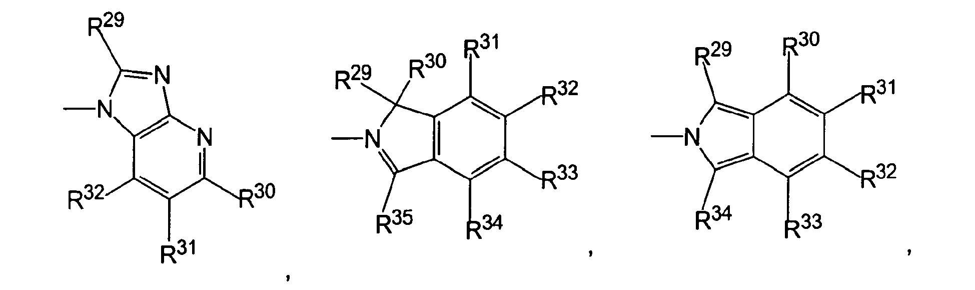 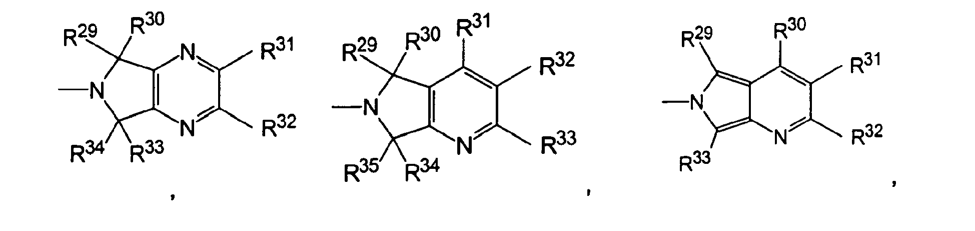 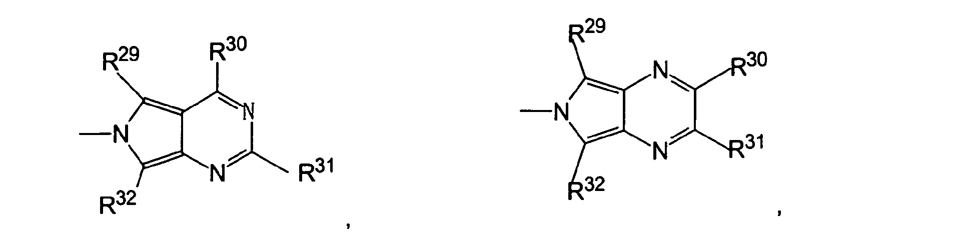 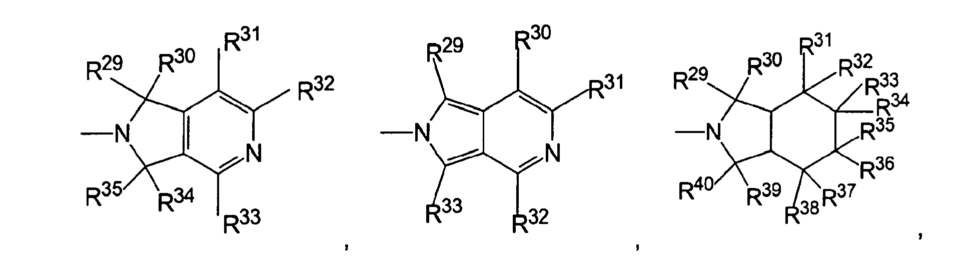 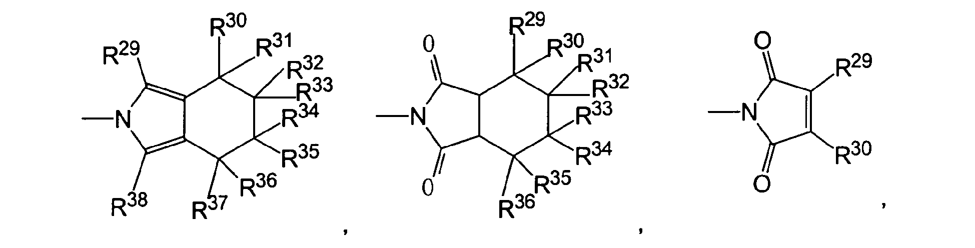 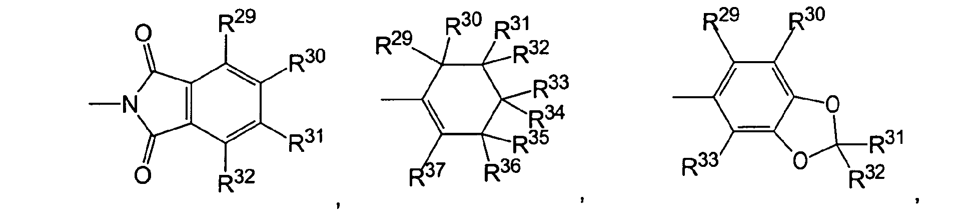 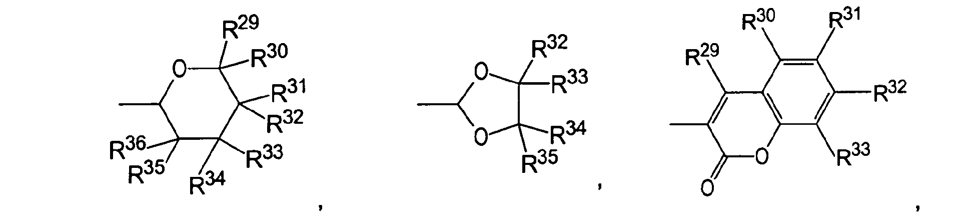 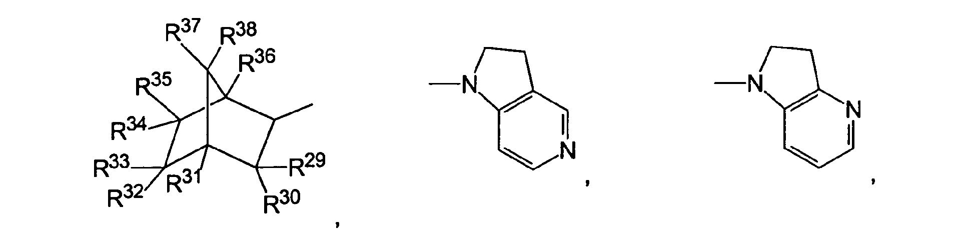 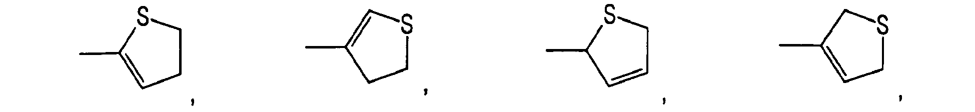 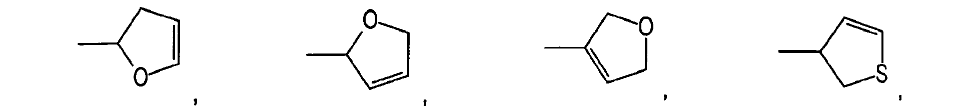 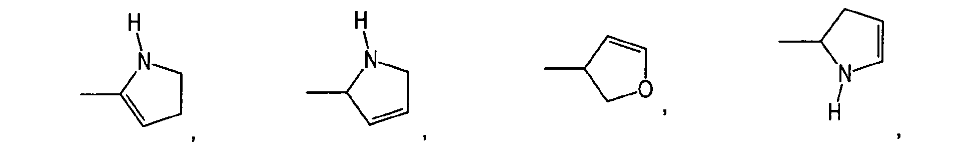 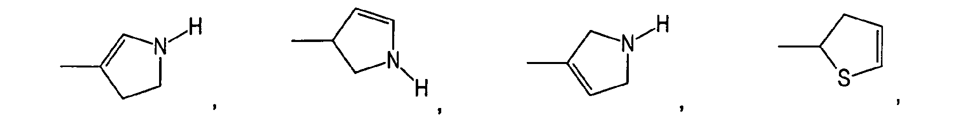 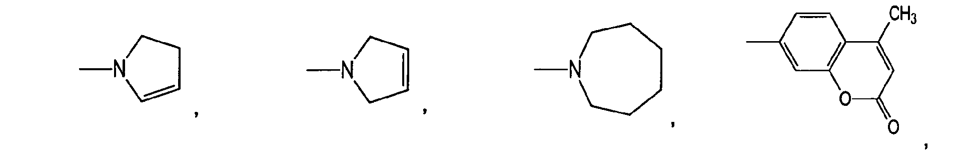 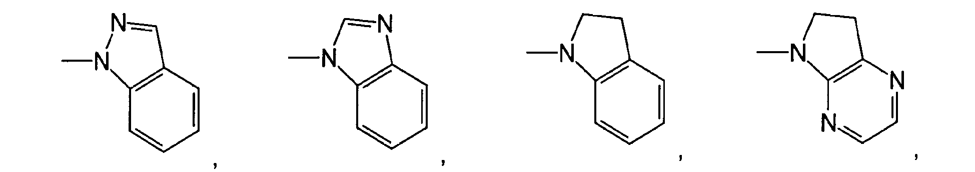 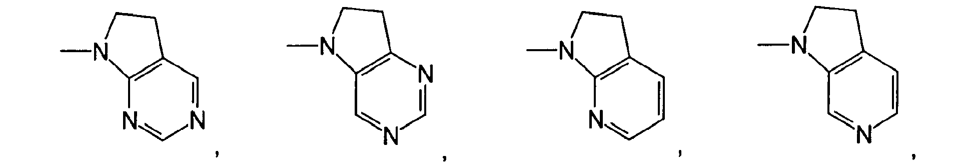 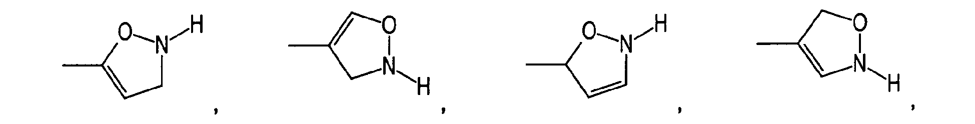 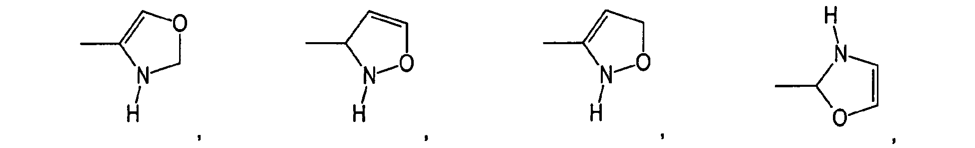 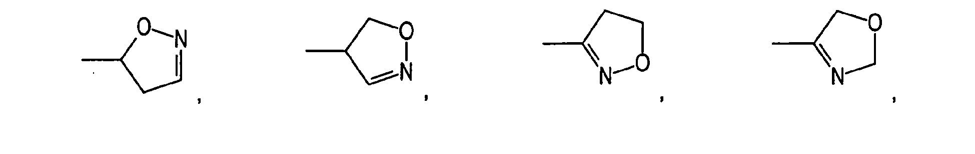 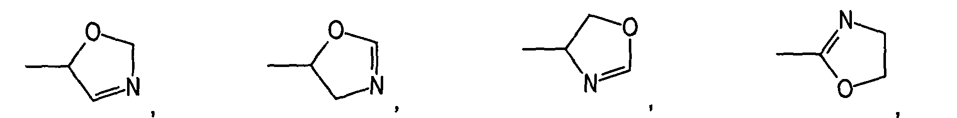 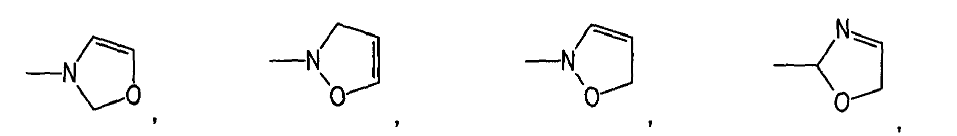 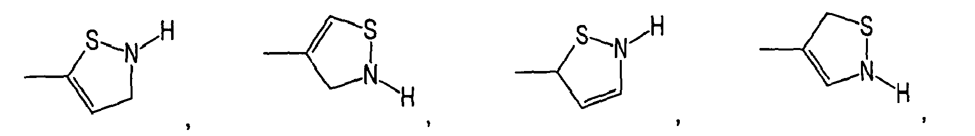 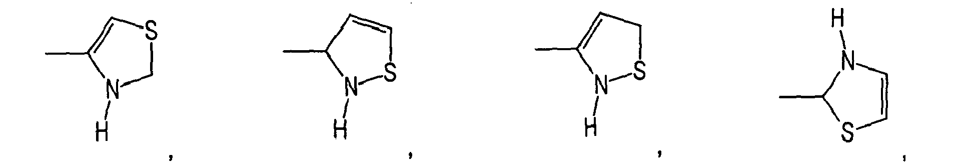 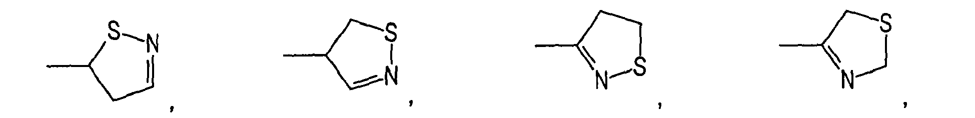 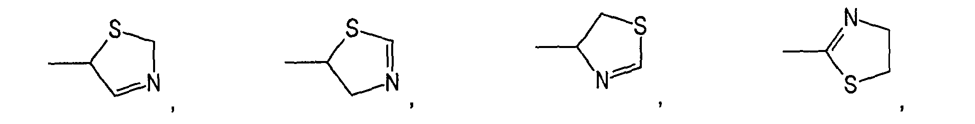 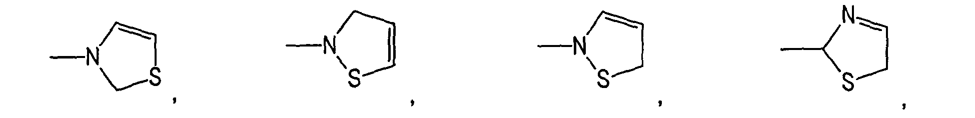 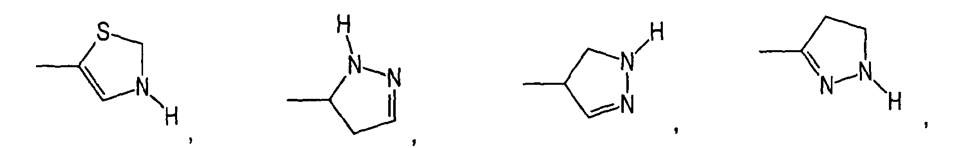 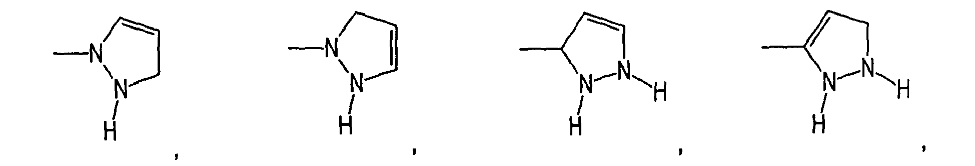 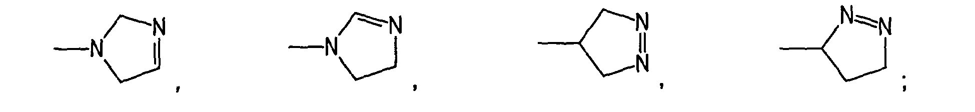
Z represents -(CH₂)ₒ-, -O-, -S-, -NH-, -CH=CH-, -C≡C-, -O-(CH₂)ᵣ-, -NH-(CH₂)ₛ-, -CO-, -CO-O-, -NH-CO-, -CO-NH-;
wherein o, r and s are independently from each other integers from 1 - 6;
**R¹⁷** - **R⁴⁴** can be selected independently from -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, **-**Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)_{2,} -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H_{5,} -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -NH-C(=NH)-NH-cyclo-C₃H₅, -NH-C(=NH)-NH[CH(CH₃)₂], -NH-C(=NH)-NH[C(CH₃)₃], -NH-C(=NH)-N(CH₃)₂, -NH-C(=NH)-N(C₂H₅)_{2,} -NH-C(=NH)-N(C₃H₇)_{2,} -NH-C(=NH)-N(cyclo-C₃H₅)₂, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃; -CH₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂I, -CH₂-CH₂OH, -CH₂-CH(Cl)-CH₃, -CH₂-CH(Br)-CH₃, -CH₂-CH(I)-CH₃, -CH₂-CH(OH)-CH₃, -CH₂-CH(NH₂)-CH₃, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁,-CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃,-CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, (CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆CH=CH-CH₃, -C₂H₄-CH=CH-C₂H₅, -CH₂-CH=CH-C₃H₇, -CH=CH-C₄H₉, -C₃H₆C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃), -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C=C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C=CH, -CH(CH₃)-CH=CH-CH=CH₂, -CH=CH-CH₂-C(CH₃)=CH₂, -CH(CH₃)-C=C-CH₃-CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, **-**CH(CH₃)-C≡CH, **-**C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, -CH=CH-Ph, -Ph, -CH₂-Ph;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

2. Compounds according to claim 1, wherein **X** represents =N-O-(CO)-R¹⁶ and R¹⁶ has the meaning as defined in claim 1

3. Compounds according to claim 1 or 2, wherein **R¹** to **R⁴** represent independently from each other -H, -F, -Cl, -Br, -CH₃, -CH₂F, -CF₃, -CH₂Cl, -CH₂Br, -CH₂-CH₂F, -CH₂-CF₃, -CH₂-CH₂Cl, -CH₂-CH₂Br, -CH₂-CH₂OH, -CH₂-CH(OH)-CH₃, -CH₂-CH(NH₂)-CH₃, cyclo-C₃H₅, cyclo-C₄H₇, cyclo-C₅H₉, cyclo-C₆H₁₁, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂C₂H₅, -CH₂-C(CH₃)₃,-CH(C₂H₅)₂, -C₂H₄-CH(CH₃)₂, -C₆H₁₃, -C₇H₁₅, -C₈H₁₇, -C₃H₆CH(CH₃)₂, -C₂H₄-CH(CH₃)-C₂H₅, -CH(CH₃)-C₄H₉, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH(CH₃)-CH=CH, -CH=C(CH₃)₂,

4. Compounds according to any one of claims 1 - 3 for use as pharmaceutically active agent in medicine.

5. Compounds according to any one of claims 1 - 4 for use as inhibitor of the Polo-like kinase 1.

6. Use of a compound according to any one of claims 1 - 5 for manufacturing a pharmaceutical composition for treatment or prophylaxis of cancer and for treatment or prophylaxis of inflammatory diseases.

7. Use according to claim 6, wherein the cancer is selected from the group comprising: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

8. Use according to claim 6, wherein the inflammatory disease is selected from the group comprising: algal disorders, protothecosis, bacterial disorders, abscessation, bacterial meningitis, idiopathic inflammatory disorders, eosinophilic meningoencephalitis, feline polioencephalomyelitis, granulomatous meningoencephalomyelitis, meningitis, steroid responsive meningitis-arteritis, miscellaneous meningitis, meningoencephalitis, meningoencephalitis in greyhounds, necrotizing encephalitis, pug dog encephalitis, pyogranulomatous meningoencephalomyelitis, shaker dog disease, mycotic diseases of the CNS, parasitic encephalomyelitis, prion protein induced diseases, feline spongiform encephalopathy, protozoal encephalitis-encephalomyelitis, toxoplasmosis, neosporosis, sarcocystosis, encephalitozoonosis, trypanosomiasis, acanthamebiasis, babesiosis, leishmaniasis, rickettsial disorders, rocky mountain spotted fever, canine ehrlichiosis, salmon poisoning, viral disorders, aujeszky's disease, borna disease, canine herpes virus encephalomyelitis, canine distemper encephalomyelitis, canine distemper encephalomyelitis in immature animals, multifocal distemper encephalomyelitis in mature animals, old dog encephalitis, chronic relapsing encephalomyelitis, post-vaccinal canine distemper encephalitis, feline immunodeficiency virus, feline infectious peritonitis, feline leukemia virus, infectious canine hepatitis, La Crosse virus encephalitis, parvovirus encephalitis, rabies, post-vaccinal rabies, tick-borne encephalitis in dogs, rheumatoid arthritis, scleroderma, lupus, polymyositis, dermatomyositis, psoriatic arthritis, ankylosing spondylitis, Reiters's syndrome, juvenile rheumatoid arthritis, bursitis, tendinitis, fibromyositis, vasculitis, autoantibodies in vasculitis, microscopic polyangiitis, giant cell arteritis, Takayasu's arteritis, vasculitis of the central nervous system, thromboangiitis obliterans, vasculitis secondary to bacterial, fungal, and parasitic infection, vasculitis and rheumatoid arthritis, vasculitis in systemic lupus erythematosus, vasculitis in the idiopathic inflammatory myopathies, relapsing polychondritis, systemic vasculitis in sarcoidosis, vasculitis and malignancy, drug-induced vasculitis, acute suppurative otitis media, bullous myringitis, granular myringitis, and chronic suppurative otitis media, which can manifest as mucosal disease, cholesteatoma, dermatoses, urticaria, spongiotic dermatitis, allergic contact dermatitis, irritant contact dermatitis, atopic dermatitis, erythemal multiforme, Stevens-Johnson syndrome, toxic epidermal necrolysis, chronic inflammatory dermatoses, psoriasis, lichen planus, discoid lupus erythematosus, acne vulgaris, ankylosing spondylitis, Behcet's disease, drug or hypersensitivity reaction, interstitial nephritis, juvenile rheumatoid arthritis, Kawasaki's disease, multiple sclerosis, psoriatic arthritis, Reiter's syndrome, relapsing polychondritis, sarcoidosis, Sjogren's syndrome, systemic lupus erythematosus, ulcerative colitis, vasculitis, vitiligo, Vogt Koyanagi Harada syndrome, acute multifocal placoid pigmentary epitheliopathy, acute retinal necrosis, birdshot choroidopathy, Fuch's heterochromic cyclitis, glaucomatocyclitic crisis, lens-induced uveitis, multifocal choroiditis, pars planitis, serpiginous choroiditis, sympathetic ophthalmia, trauma, gastroesophageal reflux disease, pediatric laryngitis, acute laryngeal infections of adults, chronic diseases, diseases connected with overexpression /overproduction of the protein amyloid A, such as arthritides, rheumatoid arthritis, asthma, bleeding disorders, chronic inflammatory lung diseases, atherosclerosis, kidney inflammation, psoriasis, allergies, Crohn's disease, ischemia / reperfusion injury, endotoxemic liver injury, inflammatory bowel disease, tuberculosis, chronic infections, familial Mediterranean fever, interstitial cystitis and skin sunburn, inflammatory diseases caused by herpes viruses, inflammatory diseases caused by bacteria, ureaplasma urealyticum, mycoplasma pneumoniae, chlamydia pneumoniae, C. pneumoniae, helicobacter pylori, inflammatory diseases caused by prions, inflammaotory diseases caused by fungi like histoplasmosis, coccidioidomycosis, candidiasis, aspergillosis, sporotrichosis, blastomycosis, inflammaotory diseases caused by spirochetes, leprosy, proprionobacterium, syphilis, Whipple's disease, leptospirosis, brucellosis, lyme disease, inflammaotory diseases caused by parasites, toxoplasmosis, acanthameba, toxocariasis, cysticercosis, onchocerciasis and allergic, immune and idiopathic disorders.

9. Pharmaceutical composition comprising at least one compound according to claim 1 as an active ingredient, together with at least one pharmaceutically acceptable carrier, excipient and/or diluent.
